(19) Europäisches Patentamt
European Patent Office
Office européen des brevets

(11) Veröffentlichungsnummer: **0 353 674 B1**

# (12) EUROPÄISCHE PATENTSCHRIFT

(45) Veröffentlichungstag der Patentschrift: **27.04.94**

(21) Anmeldenummer: **89114056.8**

(22) Anmeldetag: **29.07.89**

Die Akte enthält technische Angaben, die nach dem Eingang der Anmeldung eingereicht wurden und die nicht in dieser Patentschrift enthalten sind.

(51) Int. Cl.5: **C07D 233/60**, A01N 43/50, C07D 235/06, C07D 409/12, C07D 417/12, C07D 401/12, C07D 413/12, C07D 403/12, A01N 43/56, A01N 43/76, A01N 43/78

## (54) Phenoxyalkylsubstituierte Heteroaromaten, Verfahren zu ihrer Herstellung und ihre Verwendung zur Bekämpfung von Schädlingen.

(30) Priorität: **05.08.88 DE 3826682**
**05.08.88 DE 3826681**

(43) Veröffentlichungstag der Anmeldung:
**07.02.90 Patentblatt 90/06**

(45) Bekanntmachung des Hinweises auf die Patenterteilung:
**27.04.94 Patentblatt 94/17**

(84) Benannte Vertragsstaaten:
**BE CH DE ES FR GB IT LI NL**

(56) Entgegenhaltungen:
**EP-A- 0 069 848**
**EP-A- 0 092 706**
**EP-A- 0 132 606**
**EP-A- 0 239 047**
**EP-A- 0 286 006**
**EP-A- 0 289 919**
**DE-A- 3 408 528**
**JP-A- 5 998 083**
**JP-A-57 175 177**
**JP-A-57 175 179**

(73) Patentinhaber: **BASF Aktiengesellschaft**
**Carl-Bosch-Strasse 38**
**D-67063 Ludwigshafen(DE)**

(72) Erfinder: **Leyendecker, Joachim, Dr.**
**Stahlbuehlring 79**
**D-6802 Ladenburg(DE)**
Erfinder: **Neubauer, Hans-Juergen, Dr.**
**Mozartstrasse 6**
**D-4400 Muenster-Hiltrup(DE)**
Erfinder: **Kardorff, Uwe, Dr.**
**D 3,4**
**D-6800 Mannheim 1(DE)**
Erfinder: **Kuenast, Christoph, Dr.**
**Salierstrasse 2**
**D-6701 Otterstadt(DE)**
Erfinder: **Hofmeister, Peter, Dr.**
**Bernard-Humblot-Strasse 12**
**D-6730 Neustadt(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

Erfinder: **Krieg, Wolfgang, Dr.**
**Saarstrasse 17**
**D-6721 Weingarten(DE)**

## Beschreibung

Die vorliegende Erfindung betrifft neue phenoxyalkylsubstituierte Heteroaromaten der allgemeinen Formeln Ia und Ib

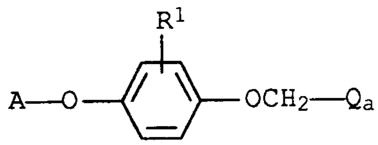

(Ia),

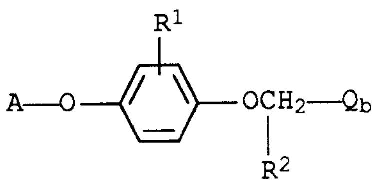

(Ib),

in denen die Substituenten die folgende Bedeutung haben:

A ein gegebenenfalls substituierter heteroaromatischer Rest der Formeln Va bis Ve

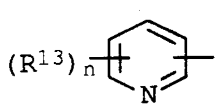

(Va)

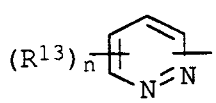

(Vb)

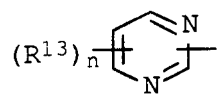

(Vc)

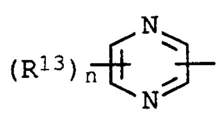

(Vd)

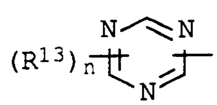

(Ve)

in denen

$R^{13}$ gleich oder verschieden ist und für Wasserstoff, Halogen, $C_1$-$C_4$-Alkyl, $C_1$-$C_3$-Halogenalkyl, $C_1$-$C_4$-Alkoxy, $C_1$-$C_3$-Halogenalkoxy, $C_3$-$C_6$-Cycloalkyl, Cyano oder Nitro steht und

n eine Zahl von 1 bis 4 bedeutet;

$R^1$ Wasserstoff, Halogen oder $C_1$-$C_3$-Alkyl,

$R^2$ Wasserstoff oder $C_1$-$C_4$-Alkyl,

$Q_a$ ein unsubstituierter oder substituierter Azolrest der Formeln IIa bis IIe

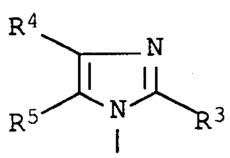

(IIa)

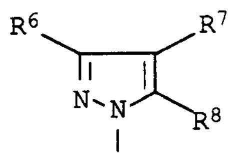

(IIb)

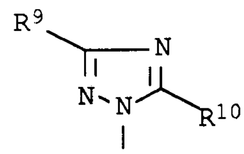

(IIc)

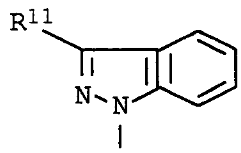

(IId)

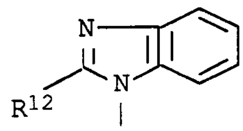

(IIe)

in denen

$R^3$ bis $R^{12}$ Wasserstoff, Halogen, $C_1$-$C_8$-Alkyl, $C_1$-$C_4$-Halogenalkyl, $C_1$-$C_4$-Alkoxy, $C_3$-$C_{10}$-Cycloalkyl oder Aryl, welches gegebenenfalls ein- bis dreifach durch Halogen, $C_1$-$C_8$-Alkyl, $C_1$-$C_8$-Alkoxy, $C_1$-$C_4$-Halogenalkyl, $C_1$-$C_3$-Halogenalkoxy substituiert ist, bedeuten,

$Q_b$ ein gegebenenfalls substituierter Fünfringheteroaromat ausgewählt aus der Gruppe Thien-2-yl, Thien-3-yl, Thiazol-2-yl, Thiazol-4-yl, Thiazol-5-yl, Oxazol-2-yl, Oxazol-4-yl, Oxazol-5-yl, Imidazol-2-yl, Imidazol-4-yl, Imidazol-5-yl, Isothiazol-3-yl, Isothiazol-4-yl, Isothiazol-5-yl, Pyrazol-3-yl, Pyrazol-4-yl, Isoxazol-3-yl, Isoxazol-5-yl, 1,3,4-Thiadiazol-2-yl, 1,3,4-Oxadiazol-2-yl, 1,2,4-Triazol-3-yl und 1,2,4-Thiadiazol-3-yl, wobei dieser Rest ein- oder mehrfach durch Halogen, $C_1$-$C_8$-Alkyl, $C_2$-$C_8$-Alkenyl, $C_1$-$C_4$-Halogenalkyl, $C_1$-$C_8$-Alkoxy, $C_1$-$C_8$-Alkylthio, $C_2$-$C_8$-Alkoxyalkyl, oder $C_3$-$C_8$-Cycloalkyl substituiert sein kann,

ausgenommen diejenigen Verbindungen Ib, in denen A für gegebenenfalls substituiertes Pyridin-2-yl steht, wenn $R^2$ Methyl und $Q_b$ ein 5-Alkoxy-substituiertes 1,3,4-Thiadiazol-2-yl bedeutet.

Außerdem betrifft die Erfindung die Herstellung der Verbindungen Ia und Ib, Schädlingsbekämpfungsmittel, die die Verbindungen Ia und Ib als Wirkstoffe enthalten, sowie ein Verfahren zur Bekämpfung von Schädlingen.

In der japanischen Patentanmeldung JP 55/28923 sind N-substituierte Azole als fungizide Wirkstoffe beschrieben. Außerdem sind aus EP-A-132 606 N-substituierte Azole als insektizide und akarizide Wirkstoffe bekannt, deren Wirkung jedoch, insbesondere bei niedrigen Aufwandmengen, zu wünschen übrig läßt.

In EP-A-92 706, DE-A-34 08 528 und den japanischen Anmeldungen JP 57/175177, JP 57/175179 und JP 59/98083 werden phenoxyalkylsubstituierte Heteroaromaten als herbizide Wirkstoffe beschrieben; einen Hinweis auf insektizide Wirkung findet man in diesen Schriften nicht.

Außerdem sind aus der EP-A-239 047 und der EP-A-286 006 Phenoxyphenoxymethyl- und Pyridyloxyphenoxymethylthiadiazole mit insektizider und akarizider Wirkung bekannt.

Der Erfindung lag daher die Aufgabe zugrunde, neue phenoxyalkylsubstituierte Heteroaromaten mit verbesserter Wirkung bereitzustellen.

Demgemäß wurden die eingangs definierten neuen phenoxyalkylsubstituierten Heteroaromaten sowie Verfahren zu deren Herstellung gefunden. Ferner wurde gefunden, daß sich die Verbindungen Ia und Ib hervorragend zur Bekämpfung von Schädlingen eignen.

Die Verbindungen Ia und Ib sind nach folgender Methode erhältlich:

Die Umsetzung erfolgt zwischen einem Phenol III und einem N-Methylazol IVa oder einem Fünfringheteroaromaten IVb in Gegenwart einer Base im Temperaturbereich von (-20) bis 250 °C, vorzugsweise von 20 bis 120 °C nach folgender Reaktionsgleichung:

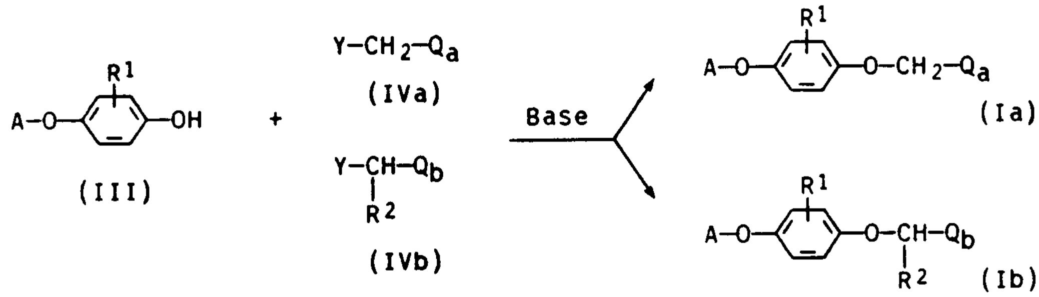


Anstelle des Phenols III plus Base kann auch das Phenolatanion von III direkt mit IVa bzw. IVb umgesetzt werden. In diesem Fall liegen die Reaktionstemperaturen bevorzugt bei (-20) bis 120, insbesondere (-20) bis 80 °C.

In den obigen Gleichungen bedeutet der Rest Y eine übliche Abgangsgruppe, wie beispielsweise einen Sulfonsäurerest oder ein Halogen. Unter den Sulfonsäureresten sind Methansulfonyl, Trifluormethansulfonyl und p-Toluolsulfonyl, unter den Halogenen sind Chlor und Brom bevorzugt; besonders bevorzugt wird Chlor.

Die Phenole III sind zum Teil aus CH-A-652 714, Res. Discl. 214, 32 (1982) und DE-A-33 20 534 bekannt, oder können nach den dort beschriebenen Methoden hergestellt werden.

Die N-Methylazole IVa sind zum Teil aus Heterocycles 24, 2233-2237 (1986) bekannt oder können nach der dort beschriebenen Methode gemäß folgender Reaktionsgleichung hergestellt werden:

$$H\text{-}Q_a \xrightarrow{(CH_2O)_3} HO\text{-}CH_2\text{-}Q_a \xrightarrow[-H_2O]{HY} Y\text{-}CH_2\text{-}Q_a$$

(IVa)

Die Heteroaromaten IVb sind entweder bekannt und teilweise kommerziell erhältlich oder lassen sich nach allgemein bekannten chemischen Verfahren herstellen. Verfahren zur Herstellung von Thiophenderivaten finden sich beispielsweise in Comprehensive Heterocyclic Chemistry, A.R. Katritzky und C.W. Rees, Vol. 4, S. 863 ff, Pergamon Press 1984; von Furanderivaten z.B. in DE-A-3 514 384, DE-A-3 546 371 oder Advances in Heterocyclic Chemistry, Vol. 30, S. 167 ff, 1982; von Pyrrolderivaten z. B. in Comprehensive Heterocyclic Chemistry, A.R. Katritzky und C.W. Rees, vol. 4, S. 313 ff, Pergamon Press, 1984; von Thiazolderivaten, Oxazolderivaten, Isothiazolderivaten, Thiadiazolderivaten und Oxadiazolderivaten z. B. in Comprehensive Heterocyclic Chemistry, A.R. Katritzky und C.W. Rees, Vol. 6, S. 235, 177, 131, 447, 365 ff, Pergamon Press, 1984; von Imidazolderivaten z. B. in Advances in Heterocyclic Chemistry, Vol. 27, S. 242 ff, 1980; von Pyrazolderivaten z. B. in Heteroaromatic Nitrogen Compounds, The Azoles, S. 31 ff, Cambridge University Press, 1976; von Triazolderivaten z. B. in Comprehensive Heterocyclic Chemistry, A.R. Katritzky und C.W. Rees, Vol. 5, S. 669 ff, Pergamon Press, 1984; von Isoxazolderivaten z. B. in DE-A-2 549 962 und DE-A-2 754 832.

Man setzt normalerweise mindestens äquivalente Mengen einer Base bezogen auf III, zu III und/oder IVa bzw. IVb zu, kann aber diese auch im überschuß oder gegebenenfalls auch als Lösungsmittel verwenden.

Als Basen eignen sich beispielsweise Hydroxide von Alkali- und Erdalkalimetallen wie Natriumhydroxid, Kaliumhydroxid und Calciumhydroxid. Alkoholate von Alkali- und Erdalkalimetallen wie Natriummethylat, Natriumethylat, Calciummethanolat oder Kalium-tert.-butylat; Alkali- oder Erdalkalimetallhydride wie Natriumhydrid, Kaliumhydrid oder Calciumhydrid; Alkali-oder Erdalkalicarbonate wie Natriumcarbonat, Kaliumcarbonat oder Calciumcarbonat; aliphatische Amine wie Dimethylamin, Triethylamin oder Diisopropylamin; heterocyclische Amine wie Piperidin, Piperazin oder Pyrrolidin; aromatische Amine wie Pyridin oder Pyrrol und gegebenenfalls auch Alkyllithium-Verbindungen wie n-Butyllithium.

Die Umsetzung wird zweckmäßigerweise in einem Lösungsmittel oder Verdünnungsmittel durchgeführt. Hierzu eignen sich beispielsweise aliphatische Kohlenwasserstoffe wie n-Pentan, n-Hexan, das Hexan-Isomerengemisch und Petrolether; aromatische Kohlenwasserstoffe wie Benzol, Toluol, die Xylole und deren Isomerengemische, Benzin; Alkohole wie Methanol, Ethanol, n-Propanol und Isopropanol; Ether wie Diethyl-, Di-n-butylether, Methyl-tert.-butylether, Tetrahydrofuran und Dioxan; Ketone wie Aceton, Methylethylketon und Methylisopropylketon; Nitrile wie Acetonitril und Propionitril; aprotische dipolare Lösungsmittel wie Dimethylformamid, Dimethylsulfoxid oder Pyridin. Auch Gemische dieser Stoffe können als Lösungs- und

Verdünnungsmittel verwendet werden.

Zur Herstellung der erfindungsgemäßen Verbindungen I nach der vorstehend beschriebenen Methode setzt man die Ausgangsstoffe überlicherweise in stöchiometrischem Verhältnis ein. Ein überschuß des einen oder anderen kann in Einzelfällen aber durchaus vorteilhaft sein.

Die Umsetzungen verlaufen gewöhnlich oberhalb von -20 °C mit ausreichenden Geschwindigkeiten. 120 °C müssen im allgemeinen nicht überschritten werden. Da sie in einigen Fällen unter wärmeentwicklung verlaufen, kann es von Vorteil sein, Kühlmöglichkeiten vorzusehen.

Die Reaktionsgemische werden in üblicher Weise aufgearbeitet, z.B. durch Versetzen mit Wasser, Trennen der Phasen und Säulenchromatographie. Die neuen Verbindungen der Formel I fallen teilweise in Form farbloser oder schwach bräunlich gefärbter, zäher Öle an, die sich durch längeres Erwärmen unter vermindertem Druck auf mäßig erhöhte Temperatur ("Andestillieren") von den letzten flüchtigen Anteilen befreien und auf diese Weise reinigen lassen. Erhält man die Verbindungen der Formeln Ia bzw. Ib in kristalliner Form, so kann ihre Reinigung durch Umkristallisieren erfolgen.

Die Substituenten in Formel I haben im einzelnen folgende Bedeutungen:

A ein gegebenenfalls substituierter heteroaromatischer Rest der Formeln Va bis Ve

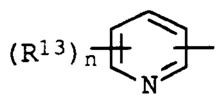
(Va)

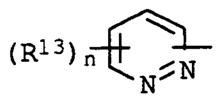
(Vb)

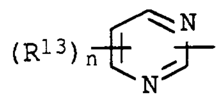
(Vc)

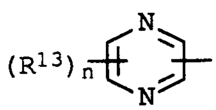
(Vd)

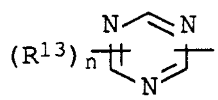
(Ve)

in denen

$R^{13}$ gleich oder verschieden ist und für Wasserstoff, Halogen, $C_1$-$C_4$-Alkyl, $C_1$-$C_3$-Halogenalkyl, $C_1$-$C_4$-Alkoxy, $C_1$-$C_3$-Halogenalkoxy, $C_3$-$C_6$-Cycloalkyl, Cyano oder Nitro steht und n eine Zahl von 1 bis 4 bedeutet,

wobei als Heteroaromaten Pyridin-2-yl, Pyridin-3-yl, Pyridin-4-yl, Pyrimidin-2-yl, Pyrimidin-4-yl, Pyrimidin-5-yl, Pyridazin-3-yl, Pyridazin-4-yl, Pyrazin-2-yl, Pyrazin-3-yl, 1,3,5-Triazin-2-yl, und insbesondere Pyridin-2-yl, Pyrimidin-2-yl, Pyrimidin-4-yl, Pyrimidin-5-yl, Pyridazin-3-yl und Pyrazin-2-yl bevorzugt sind und als Reste $R^{13}$ unabhängig voneinander

- Wasserstoff,
- Halogen wie Fluor, Chlor und Brom, insbesondere Fluor und Chlor,
- unverzweigtes oder verzweigtes $C_1$-$C_4$-Alkyl, wie Methyl, Ethyl, Propyl, Butyl, insbesondere unverzweigtes Alkyl wie Methyl und Ethyl oder verzweigtes Alkyl wie Isopropyl, Isobutyl und sec.-Butyl,
- unverzweigtes oder verzweigtes $C_1$-$C_3$-Halogenalkyl, insbesondere Fluor- und Chloralkyl wie Fluormethyl, Difluormethyl, Trifluormethyl, Pentafluorethyl, 2,2,2,-Trifluoroethyl, Chlormethyl, Dichlormethyl, Trichlormethyl und 2,2,2-Trichloroethyl,
- unverzweigtes oder verzweigtes $C_1$-$C_4$-Alkoxy, insbesondere Methoxy, Ethoxy und Isopropyloxy,
- unverzweigtes oder verzweigtes $C_1$-$C_3$-Halogenalkoxy, insbesondere Fluor- und Chloralkoxy wie Trifluormethoxy, Trichlormethoxy, 1,1,2,2-Tetrafluoroethoxy und Pentafluoroethoxy,
- $C_3$-$C_6$-Cycloalkyl wie Cyclopropyl, Cyclobutyl, Cyclopentyl und Cyclohexyl, insbesondere Cyclopropyl,
- Cyano,
- Nitro,

in Frage kommen;

$R^1$

- Wasserstoff,

- Halogen wie Fluor, Chlor und Brom, bevorzugt Fluor und Chlor,
- unverzweigtes oder verzweigtes $C_1$-$C_3$-Alkyl wie Methyl, Ethyl, Propyl und Isopropyl,

$R^2$
- Wasserstoff,
- $C_1$-$C_4$-Alkyl wie Methyl, Ethyl, Propyl, Isopropyl, Butyl, besonders bevorzugt Methyl,

$R^3$ - $R^{12}$ unabhängig voneinander
- Wasserstoff,
- Halogen, bevorzugt Fluor und Chlor,
- unverzweigtes oder verzweigtes $C_1$-$C_8$-Alkyl, bevorzugt unverzweigtes oder verzweigtes $C_1$-$C_4$-Alkyl wie Methyl, Ethyl, Propyl, Butyl, besonders bevorzugt $C_1$-$C_2$-Alkyl wie Methyl und Ethyl,
- unverzweigtes oder verzweigtes $C_1$-$C_4$-Halogenalkyl, bevorzugt $C_1$-$C_2$-Fluor- und Chloralkyl, besonders bevorzugt Trifluormethyl und Trichlormethyl,
- unverzweigtes oder verzweigtes $C_1$-$C_8$-Alkoxy, bevorzugt unverzweigtes oder verzweigtes $C_1$-$C_4$-Alkoxy, besonders bevorzugt $C_1$-$C_2$-Alkoxy wie Methoxy und Ethoxy,
- $C_3$-$C_{10}$-Cycloalkyl, bevorzugt $C_3$-$C_6$-Cycloalkyl wie Cyclopropyl, Cyclobutyl, Cyclopentyl, Cyclohexyl, besonders bevorzugt Cyclopropyl,
- Aryl, bevorzugt Phenyl, 1-Naphthyl und 2-Naphthyl, besonders bevorzugt Phenyl,
- ein- bis dreifach durch Halogen substituiertes Aryl, bevorzugt durch Fluor oder Chlor monosubstituiertes Phenyl wie 2-Fluorphenyl, 3-Fluorphenyl, 4-Fluorphenyl, 2-Chlorphenyl, 3-Chlorphenyl, 4-Chlorphenyl,
- ein- bis dreifach durch unverzweigtes oder verzweigtes $C_1$-$C_8$-Alkyl substituiertes Aryl, bevorzugt durch unverzweigtes oder verzweigtes $C_1$-$C_4$-Alkyl monosubstituiertes Phenyl, besonders bevorzugt durch $C_1$-$C_2$-Alkyl monosubstituiertes Phenyl wie 4-Methylphenyl und 4-Ethylphenyl,
- ein - bis dreifach durch unverzweigtes oder verzweigtes $C_1$-$C_8$-Alkoxy substituiertes Aryl, bevorzugt durch unverzweigtes oder verzweigtes $C_1$-$C_4$-Alkoxy monosubstituiertes Phenyl, besonders bevorzugt durch $C_1$-$C_2$-Alkoxy monosubstituiertes Phenyl wie 4-Methoxyphenyl und 4-Ethoxyphenyl,
- ein- bis dreifach durch unverzweigtes oder verzweigtes $C_1$-$C_4$-Halogenalkoxy substituiertes Aryl, bevorzugt durch $C_1$-$C_2$-Fluor- und Chloralkoxy monosubstituiertes Phenyl, besonders bevorzugt durch Trifluormethoxy und Trichlormethoxy monosubstituiertes Phenyl, wie 4-Trifluormethoxyphenyl und 4-Trichlormethoxyphenyl.

$Q_b$ ein gegebenenfalls substituierter Fünfringheteroaromat ausgewählt aus der Gruppe Thien-2-yl, Thien-3-yl, Thiazol-2-yl, Thiazol-4-yl, Thiazol-5-yl, Oxazol-2-yl, Oxazol-4-yl, Oxazol-5-yl, Imidazol-2-yl, Imidazol-4-yl, Imidazol-5-yl, Isothiazol-3-yl, Isothiazol-4-yl, Isothiazol-5-yl, Pyrazol-3-yl, Pyrazol-4-yl, Isoxazol-3-yl, Isoxazol-5-yl, 1,3,4-Thiadiazol-2-yl, 1,3,4-Oxadiazol-2-yl, 1,2,4-Triazol-3-yl, 1,2,4-Thiadiazol-3-yl, besonders bevorzugt Thien-2-yl, Thien-3-yl, Thiazol-4-yl, Imidazol-5-yl, 1,3,4-Thiadiazol-2-yl, 1,3,4-Oxadiazol-2-yl, Isoxazol-3-yl und Isoxazol-5-yl.

Der Fünfringheteroaromat kann gegebenenfalls ein- oder mehrfach substituiert sein mit:
- Halogen, bevorzugt Fluor, Chlor und Brom, besonders bevorzugt Chlor und Brom,
- $C_1$-$C_8$-Alkyl, bevorzugt $C_1$-$C_4$-Alkyl, besonders bevorzugt Methyl, Ethyl, Isopropyl und tert.-Butyl,
- $C_2$-$C_8$-Alkenyl, bevorzugt $C_2$-$C_4$-Alkenyl, besonders bevorzugt Ethenyl, 1-Methyl-ethen-1-yl, Propen-1-yl, 2-Methylpropen-1-yl,
- $C_1$-$C_4$-Halogenalkyl, bevorzugt Fluor oder Chlor substituiertes $C_1$-$C_2$-Halogenalkyl, besonders bevorzugt Trifluormethyl, 2,2,2-Trifluorethyl,
- $C_1$-$C_8$-Alkoxy, bevorzugt $C_1$-$C_3$-Alkoxy, besonders bevorzugt Methoxy, Ethoxy, n-Propyloxy und Isopropyloxy,
- $C_1$-$C_8$-Alkylthio, bevorzugt $C_1$-$C_3$-Alkylthio, besonders bevorzugt Methylthio, Ethylthio, n-Propylthio und Isopropylthio,
- $C_2$-$C_8$-Alkoxyalkyl, bevorzugt $C_2$-$C_4$-Alkoxyalkyl, besonders bevorzugt Methoxymethyl, 1-Methoxyethyl, 2-Methoxyethyl, 1-Methoxypropyl,
- $C_3$-$C_8$-Cycloalkyl, bevorzugt $C_3$-$C_5$-Cycloalkyl, besonders bevorzugt Cyclopropyl, Cyclobutyl und Cyclopentyl.

Die phenoxyalkylsubstituierten Heteroaromaten der allgemeinen Formeln Ia bzw. Ib sind geeignet, Schädlinge aus der Klasse der Insekten, Spinnentiere und Nematoden wirksam zu bekämpfen. Sie können im Pflanzenschutz sowie auf dem Hygiene-, Vorratsschutz- und Veterinärsektor als Schädlingsbekämpfungsmittel eingesetzt werden.

Sie können daher in Verfahren zur Bekämpfung von Schädlingen eingesetzt werden, wobei man die Schädlinge und/oder die von Schädlingen freizuhaltenden Flächen und/oder Räume mit einer für Schädlinge wirksamen Menge eines phenoxyalkylsubstituierten Heteroaromaten der Formeln Ia und Ib behandelt.

Zu den schädlichen Insekten gehören aus der Ordnung der Schmetterlinge (Lepidoptera) beispielsweise Plutella maculipennis (Kohlschabe), Leucoptera coffeella (Kaffeemotte), Hyponomeuta malinellus (Apfelbaumgespinstmotte), Argyresthia conjugella (Apfelmotte), Sitotroga cerealella (Getreidemotte), Phthorimaea operculella (Kartoffelmotte), Capus reticulana (Apfelschalenwickler), Sparganothis pilleriana (Springwurm), Cacoecia murinana (Tannentriebwickler), Tortrix viridana (Eichenwickler), Cylsia ambiguella (Heu- und Sauerwurm), Evetria buoliana (Kieferntriebwickler), Polychrosis botrana (Bekreuzter Traubenwickler), Cydia pomonella (Obstmade), Laspeyresia molesta (Pfirsichtriebbohrer), Laspeyresia funebrana (Pflaumenwickler), Ostrinia nubilalis (Maiszünsler), Loxostege sticticallis (Rübenzünsler), Ephestia kuehniella (Mehlmotte), Chilo suppressalis (Reisstengelbohrer), Galleria mellonella (Wachsmotte), Malacosoma neustria (Ringelspinner), Dendrolimus pini (Kiefernspinner), Thaumotpoea ptiycampa (Pinieprozessionsspinner), Phalera bucephala (Mondfleck), Cheimatobia brumata (Kleiner Frostspanner), Hibernia defoliaria (Großer Frostspanner), Bupalus piniarius (Kiefernspanner), Hyphantria cunea (Weißer Bärenspinner), Agrotis segetum (Wintersaateule), Agrotis ypsilon (Ypsiloneule), Barathra brassicae (Kohleule), Cirphis unipuncta (Heerwurm), Prodenia litura (Baumwollraupe), Laphygma exigua (Rüben-Heerwurm), Panolis flammea (Forleule), Earias insulana (Baumwollkapselwurm), Plusia gamma (Gammaeule), Alabama argillacea (Baumwollblattwurm), Lymantria dispar (Schwammspinner), Lymantria monacha (Nonne), Pierus brassicae (Kohlweißling), Aporia crataegi (Baumweißling);

aus der Ordnung der Käfer (Coleoptera) beispielsweise Blitophaga undata (Schwarzer Rübenaaskäfer), Melanotus communis (Drahtwurm), Limonius californicus (Drahtwurm), Agriotes lineatus (Saatschnellkäfer), Agriotes obscurus (Humusschnellkäfer), Agrilus sinuatus (Birnbaum-Prachtkäfer) Meligethes aeneus (Rapsglanzkäfer), Atomaria linearis (Moosknopfkäfer), Epilachna varivestis (Mexikanischer Bohnenkäfer), Phyllopertha horticola (Junikäfer), Popillia japonica (Japankäfer), Melolontha melolontha (Feldmaikäfer), Melolontha hippocastani (Waldmaikäfer), Amphimallus solstitialis (Brachkäfer), Crioceris aspargi (Spargelhähnchen), Lema melanopus (Getreidehähnchen), Leptinotarsa decemlineata (Kartoffelkäfer), Phaedon cochleariae (Meerrettich-Blattkäfer), Phyllotreta nemorum (Kohlerdfloh), Chaetocnema tibialis (Rübenflohkäfer), Phylloides chrysocephala (Raps-Flohkäfer), Diabrotica 12-punctata (Südlicher Maiswurzelwurm), Cassida nebulosa (Nebliger Schildkäfer), Bruchus lentis (Linsenkäfer), Bruchus rufimanus (Pferdebohnenkäfer), Bruchus pisorum (Erbsenkäfer), Sitona lineatus (Liniierter Blattrandkäfer), Ortiorrhynchus salcatus (Gefurchter Lappenrüßler), Ortiorrhynchus ovatus (Erdbeerwurzelrüßler), Hylobies abietis (Großer Brauner Rüsselkäfer), Byctiscus betulae (Rebenstecher), Anthonomus pomorum (Apfelblütenstecher), Anthonomus grandis (Kapselkäfer), Ceuthorrhynchus assimilis (Kohlschotenrüßler), Ceuthorrynchus napi (Großer Kohltriebrüßler) Sitophilus granaria (Kornkäfer), Anisandrus dispar (Ungleicher Holzborkenkäfer), Ips typographus (Buchdrucker), Bastophagus piniperda (Gefruchter Waldgärtner);

aus der Ordnung der Zweiflügler (Diptera) beispielsweise Lycoria pectoralis, Mayetiola destructor (Hessenfliege), Basineura brassicae (Kohlschoten-Gallmücke), Contarinia tritici (Gelbe Weizen-Gallmücke), Haplodiplosis equestris (Sattelmücke) Tipula plaudose (Wiesenschnacke), Tipula oleracea (Kohlschnacke), Dacus cucurbitae (Melonenfliege), Dacus oleae (Olivenfliege), Ceratitis capitata (Mittelmeerfruchtfliege), Rhagoletis cerasi (Kirschfruchtfliege), Rhagoletis pomonella (Apfelmade), Anastrepha ludens (Mexikanische Fruchtfliege), Oscinella frit (Fritfliege), Phorbia coarctata (Brachfliege), Phorbia antiqua (Zwiebelfliege), Phorbia brassicae (Kleine Kohlfliege), Pegomya hysocyami (Rübenfliege), Anopheles maculipennis, Culex pipiens, Aedes aegypti (Gelbfiebermücke), Aedes vexans, Tabanus bovinus (Rinderbremse), Tipula paludose (Wiesenschnacke), Musca domestica (Stubenfliege), Fannia canicularis (Kleine Stubenfliege), Muscina stabulans, Glossina morsitans (Tsetse-Fliege), Oestrus ovis, Chrysomya macellaria, Chrysomya hominivorax, Lucilia cuprina, Lucilia sericata, Hypoderma lineata;

aus der Ordnung der Hautflügler (Hymenoptera) beispielsweise Athalia rose (Rübenblattwespe), Hoplocampa minuta (Pflaumensägewespe), Monomorium pharaonis (Pharaoameise), solenopsis geminata (Feuerameise), Atta sexdens (Blattschneiderameise);

aus der Ordnung der Wanzen (Heteroptera) beispielsweise Nezara viridula (Grüne Reiswanze), Eurygaster integriceps (Asiathische Getreidewanze), Blissus leucopterus (Chinch bug), Dysdercus cingulatus (Kapok-Wanze), Dysdercus intermedius (Baumwollwanze), Piesma quadrata (Rübenwanze), Lygus pratensis (Gemeine Wiesenwanze);

aus der Ordnung der Pflanzensauger (Homoptera) beispielsweise Perkinsiella saccharicida (Zuckerrohrzikade), Nilparvata lugens (Braune Zikade), Empoasca fabae (Kartoffelzikade), Psylla mali (Apfelblattsauger), Psylla piri (Birnblattsauger), Trialeurodes vaporariorum (Weiße Fliege), Aphis fabae (Schwarze Bohnenlaus),

Aphis pomi (Grüne Apfellaus), Aphis sambuci (Holunderblattlaus), Aphidula nasturtii (Kreuzdornblattlaus), Cerosipha gossypii (Gurkenblattlaus), Sappaphis mali (Rosige Apfellaus), Sappaphis mala (Mehlige Birnblattlaus), Dyasphis radicola (Mehlige Apfelfaltenlaus), Brachycaudus cardui (Große Pflaumenblattlaus), Brevicoryne brassicae (Kohlblattlaus), Phorodon humuli (Hopfenblattlaus), Rhopalomyzus ascalonicus (Zwiebellaus), Myzodes persicae (Grüne Pfirsichlaus), Myzus cerasi (Schwarze Sauerkirschenlaus), Dysaulacorthum pseudosolani (Gefleckte Kartofellaus), Acyrthosiphon onobrychis (Grüne Erbsenlaus), Macrosiphon rosae (Große Rosenblattlaus), Megoura viciae (Wickenlaus), Schizoneura lanuginosa (Birnenblattlaus), Pemphigus bursarius (Salatwurzellaus), Dreyfusia nordmannianae (Tanntrieblaus), Dreyfusia piceae (Weißtannenstammlaus), Adelges laricis (Rote Fichtengallenlaus), Viteus vitifolii (Reblaus);

aus der Ordnung der Termiten (Isoptera) beispielsweise Reticulitermes lucifugus, Calotermes flavicollis, Leucotermes flavipes, Termes natalensis;

aus der Ordnung der Geradflügler (Orthoptera) beispielsweise Forficula auricularia (Gemeiner Ohrwurm), Acheta domestica (Heimchen), Gryllotalpa gryllotalpa (Maulwurfsgrille), Tachycines asynamorus (Gewächshausheuschrecke), Lousta migratoria (Wanderheuschrecke), Stauronotus maroccanus (Marrokkanische Wanderheuschrecke), Schistocerca peregrina (Wanderheuschrecke), Nomadacris septemfasciata (Wanderheuschrecke), Melanoplus spretus (Felsengebirgsheuschrecke), Melanoplus femur-rubrum (Rotbeinige Heuschrecke), Blatta orientalis (Küchenschabe), Blattella germanica (Deutsche Schabe), Periplaneta americana (Amerikanische Schabe), Blabera gigantes (Riesenschabe).

Zur Klasse der Arachnoidea gehören Spinntentiere (Acarina) beispielsweise Ixodes ricinus (Holzbock), Ornithodorus moubata, Amblyomma americanum, Dermacentor silvarum, Boophilus microplusum, Tetranychus telarius, Tetranychus pacificus, Paratetranychus pilosus, Bryobia praetiosa.

Zur Klasse der Nematoden zählen beispielsweise Wurzelgallennematoden, z.B. Meloidogyne incognita, Meloidogyne hapla, Meloidogyne javanica, Zysten bildende Nematoden, z.B. Globodera rostochiensis, heterodera avenae, Heterodera glycinae, Heterodera trifolii, Stock- und Blattälchen, z.B. Ditylenchus dipsaci, Ditylenchus destructor, Pratylenchus neglectus, Pratylenchus penetrans, Pratylenchus goodeyi, Pratylenchus curvitatus sowie Tylenchorrhynchus dubius, Tylenchorhynchus claytoni, Rotylenchus robustus, Heliocotylenchus multicinctus, Radopholus similis, Belonolaimus longicaudatus, Longidorus elongatus, Trichodrus primitivus.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, Methanol, Ethanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon, Wasser, in Betracht.

Wäßrige Anwendungsformen können als Emulsionskonzentrate, Pasten oder netzbare Pulver (Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkai- und Erdalkalisalze der Dibutylnaphthalinsulfonsäure, Laurylethersulfat, Fettalkoholsulfate, fettsaure Alkali- und Erdalkali, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctylphenol, Octylphenol, Nonylphenon, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolaglykoletheracetal, Sorbitester, Lignin, Sulfitablaugen und Methylcellulose in Betracht.

Pulver-, Streu- und Stäubemittel können durch Mischen oder gemeinsames Verhamlen der wirksamen Substanzen mit einem festen Trägerstoff hergestellt werden.

Granulate z.B. Umhüllungs- und Imprägnierungs- und Homogengranulate, können durch Bindung der Wirkstoffe an feste Trägerstoffe hergestellt werden. Feste Trägerstoffe sind z.B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten im allgemeinen zwischen 0,1 und 95 Gew.% Wirkstoff, vorzugsweise zwischen 0,5 und 90 Gew.%.

Beispiele für Formulierungen sind:

I. 5 Gew.-Teile der Verbindung Nr. 1.2 werden mit 95 Gew.-Teilen feinteiligem Kaolin innig vermischt. Man erhält auf diese Weise ein Stäubemittel, das 3 Gew.% des Wirkstoffs enthält.

II. 30 Gew.-Teile der Verbindung Nr. 1.2 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

III. 10 Gew.-Teile der Verbindung Nr. 1.13 werden in einer Mischung gelöst, die aus 90 Gew.-Teilen Xylol, 6 Gew.-Teilen des Anlagerungsproduktes von 8 bis 10 Mol Ethylenoxid an 1 Mol Ölsäure-N-monoethanolamid, 2 Gew.-Teilen Calciumsalz der Dodecylbenzolsulfonsäure und 2 Gew.-Teilen des Anlagerungsproduktes von 40 Mol Ethylenoxid an 1 Mol Ricinusöl besteht.

IV. 20 Gew.-Teile der Verbindung Nr. 6.3. werden in einer Mischung gelöst, die aus 60 Gew.-Teilen Cyclohexanon, 30 Gew.-Teilen Isobutanol, 5 Gew.-Teilen des Anlagerungsproduktes von 7 Mol Ethylenoxid an 1 Mol Isooctylphenol und 5 Gew.-Teilen des Anlagerungsproduktes von 40 Mol Ethylenoxid an 1 Mol Ricinusöl besteht.

V. 80 Gew.-Teile der Verbindung Nr. 6.22 werden mit 3 Gew.-Teilen des Natriumsalzes der Diisobutylnaphthalin-alpha-sulfonsäure, 10 Gew.-Teilen des Natriumsalzes einer Ligninsulfonsäure aus einer Sulfit-Ablauge und 7 Gew.-Teilen pulverförmigem Kieselsäuregel gut vermischt und in einer Hammermühle vermahlen.

Die Wirkstoffkonzentrationen in den anwendungsfertigen Zubereitungen können in größeren Bereichen variiert werden. Im allgemeinen liegen sie zwischen 0,0001 und 10 %, vorzugsweise zwischen 0,01 und 1 %. Die Wirkstoffe können auch mit gutem Erfolg im Ultra-Low-Volume-Verfahren (ULV) verwendet werden, wobei es möglich ist, Formulierungen mit mehr als 95 Gew.-% Wirkstoff oder sogar den Wirkstoff ohne Zusätze auszubringen.

Die Aufwandmenge an Wirkstoff beträgt unter Freilandbedingungen 0,001 bis 10, insbesondere 0,1 bis 2, vorzugsweise 0,01 bis 1 kg/ha.

Zu den Wirkstoffen können Öle verschiedenen Typs, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt werden.

## Herstellungsbeispiele

### Beispiel 1

1-[4-(6-Chlor-2-pyridinyloxy)phenoxymethyl]-4,5-dichlorimidazol (Verbindung Nr. 1.2)

Zu 0,6 g 100 %igem Natriumhydrid in 20 ml trockenem Dimethylformamid tropft man bei Raumtemperatur (ca. 20 °C) 4,9 g 4-(6-Chlor-2-pyridinyloxy)phenol in 40 ml trockenem Dimethylformamid. Anschließend rührt man noch 30 Minuten bei 50 °C nach und tropft bei Raumtemperatur (ca. 20 °C) 4,2 g 1-Chlormethyl-4,5-dichlorimidazol in 20 ml trockenem Dimethylformamid zu. Man rührt 2 Stunden bei 60 °C und über Nacht bei Raumtemperatur (ca. 20 °C) nach. Zur Aufarbeitung rührt man in 200 ml Eiswasser ein und extrahiert die Lösung 3mal mit Methyl-tert.-butylether. Die organische Phase wird mit 5 %iger Natronlauge und Wasser gewaschen, getrocknet und das Lösungsmittel im Vakuum verdampft. Das erhaltene Rohprodukt wird aus Toluol umkristallisiert. Man erhält 6,6 g 1-[4-(6-Chlor-2-pyridinyloxy)phenoxymethyl]4,5-dichlorimidazol mit Fp.: 94 bis 98 °C.

Beispiel 2

1-[4-(3,6-Dimethyl-2-pyrazinyloxy)phenoxymethyl]-4,5-dichlorimidazol (Verbindung Nr. 1.13)

Zu 1,3 g 80 %igem Natriumhydrid in 30 ml trockenem Dimethylformamid tropft man bei Raumtemperatur (ca. 20 ° C) 7,8 g 4-(3,6-Dimethyl-2-pyrazinyloxy)phenol in 20 ml trockenem Dimethylformamid. Anschließend rührt man 1 Stunde bei 70 ° C nach und tropft dann 6,68 g 1-Chlormethyl-4,5-dichlorimidazolin 30 ml trockenem Dimethylformamid zu. Anschließend rührt man 8 Stunden bei 70 ° C und über Nacht bei Raumtemperatur (ca. 20 ° C) nach. Zur Aufarbeitung rührt man in 200 ml Essigester ein, wäscht 2mal mit Wasser, 4mal mit 5 %iger Natronlauge und 2mal mit gesättigter Natriumchloridlösung. Die organische Phase wird über Magnesiumsulfat getrocknet und anschließend das Lösungsmittel im Vakuum verdampft. Das erhaltene Rohprodukt wird mit n-Hexan zur Kristallisation gebracht. Man erhält 8,9 g 1-[4-(3,6-Dimethyl-2-pyrazinyloxy)phenoxymethyl]-4,5-dichlorimidazol mit Fp.: 84 bis 85 ° C.

Entsprechend dieser Herstellvorschriften können die in den folgenden Tabellen 1 bis 5 beschriebenen Verbindungen Iaa bis Iae hergestellt werden. Die Substitutionsstelle am Heteroaromaten ist jeweils durch einen Punkt gekennzeichnet.

Die ohne Angabe physikalischer Kenngrößen in den nachstehenden Tabellen 1 bis 5 aufgeführten Verbindungen Iaa bis Iae können aus entsprechenden Vorprodukten ohne weiteres erhalten werden und lassen eine gleichartige Wirkung erwarten.

Tabelle 1

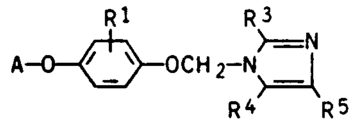

(Iaa)

| Verbindung Nr. | A | $R^1$ | $R^3$ | $R^4 = R^5$ | phys. Daten Fp. [°C] |
|---|---|---|---|---|---|
| 1.1 | 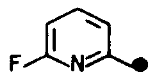 | H | H | Cl | 90 - 95 |
| 1.2 | 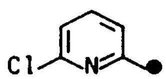 | H | H | Cl | 94 - 98 |
| 1.3 | 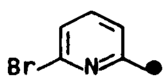 | H | H | Cl | 99 - 101 |
| 1.4 | 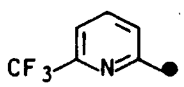 | H | H | Cl | 58 - 60 |
| 1.5 | 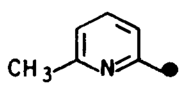 | H | H | Cl | |
| 1.6 | 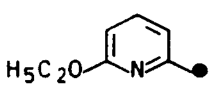 | H | H | Cl | |
| 1.7 | 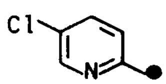 | H | H | Cl | 94 - 97 |
| 1.8 | 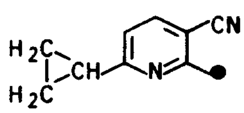 | H | H | Cl | 149 - 154 |
| 1.9 | 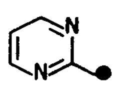 | H | H | Cl | 154 - 175 |
| 1.10 | 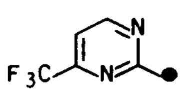 | H | H | Cl | 92 - 98 |
| 1.11 | 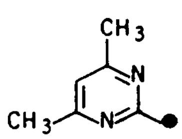 | H | H | Cl | 109 - 122 |

Tabelle 1 (Fortsetzung)

| Verbindung Nr. | A | $R^1$ | $R^3$ | $R^4 = R^5$ | phys. Daten Fp. [°C] |
|---|---|---|---|---|---|
| 1.12 | 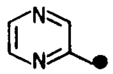 | H | H | Cl | 79 - 90 |
| 1.13 | 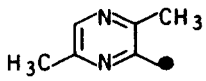 | H | H | Cl | 84 - 85 |
| 1.14 | 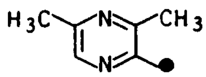 | H | H | Cl | |
| 1.15 | 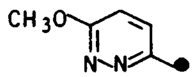 | H | H | Cl | 117- 124 |
| 1.16 | 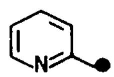 | H | H | Cl | |
| 1.17 | 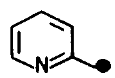 | H | H | $CH_3$ | |
| 1.18 | 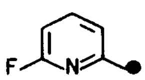 | H | H | $CH_3$ | |
| 1.19 | 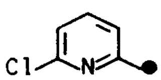 | H | H | $CH_3$ | |
| 1.20 | 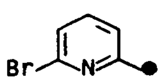 | H | H | $CH_3$ | |
| 1.21 | 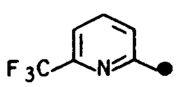 | H | H | $CH_3$ | |
| 1.22 | 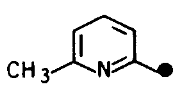 | H | H | $CH_3$ | |
| 1.23 | 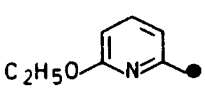 | H | H | $CH_3$ | |
| 1.24 | 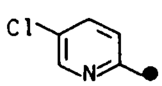 | H | H | $CH_3$ | |

Tabelle 1 (Fortsetzung)

| Verbindung Nr. | A | $R^1$ | $R^3$ | $R^4 = R^5$ | phys. Daten Fp. [°C] |
|---|---|---|---|---|---|
| 1.25 | 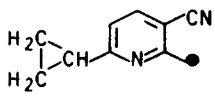 | H | H | $CH_3$ | |
| 1.26 | 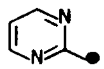 | H | H | $CH_3$ | |
| 1.27 | 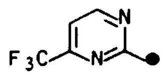 | H | H | $CH_3$ | |
| 1.28 | 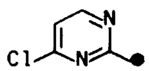 | H | H | $CH_3$ | |
| 1.29 | 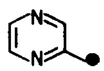 | H | H | $CH_3$ | |
| 1.30 | 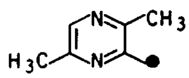 | H | H | $CH_3$ | |
| 1.31 | 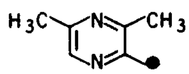 | H | H | $CH_3$ | |
| 1.32 | 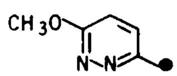 | H | H | $CH_3$ | |
| 1.33 | 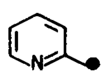 | H | H | H | |
| 1.34 | 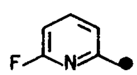 | H | H | H | |
| 1.35 | 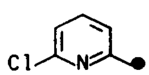 | H | H | H | |
| 1.36 | 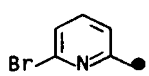 | H | H | H | |
| 1.37 | 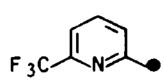 | H | H | H | |
| 1.38 | 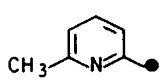 | H | H | H | |

Tabelle 1 (Fortsetzung)

| Verbindung Nr. | A | $R^1$ | $R^3$ | $R^4$ = $R^5$ | phys. Daten Fp. [°C] |
| --- | --- | --- | --- | --- | --- |
| 1.39 | 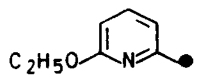 | H | H | H | |
| 1.40 | 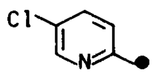 | H | H | H | |
| 1.41 | 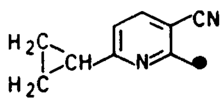 | H | H | H | |
| 1.42 | 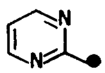 | H | H | H | |
| 1.43 | 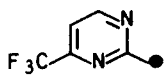 | H | H | H | |
| 1.44 | 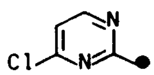 | H | H | H | |
| 1.45 | 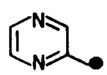 | H | H | H | |
| 1.46 | 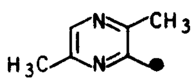 | H | H | H | |
| 1.47 | 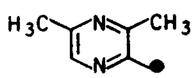 | H | H | H | |
| 1.48 | 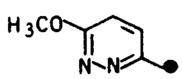 | H | H | H | |
| 1.49 | 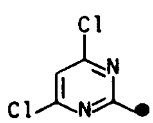 | H | H | Cl | |
| 1.50 | 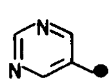 | H | H | Cl | |
| 1.51 | 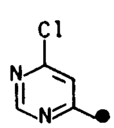 | H | H | Cl | |

Tabelle 2

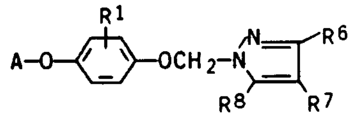

(Iab)

| Verbindung Nr. | A | R1 | R6 | R7 | R8 | phys. Daten |
|---|---|---|---|---|---|---|
| 2.1 | 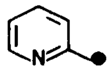 | H | H | H | H | |
| 2.2 | 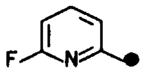 | H | H | H | H | |
| 2.3 | 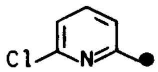 | H | H | H | H | |
| 2.4 | 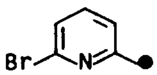 | H | H | H | H | |
| 2.5 | 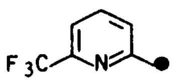 | H | H | H | H | |
| 2.6 | 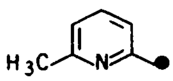 | H | H | H | H | |
| 2.7 | 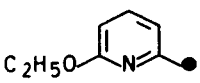 | H | H | H | H | |
| 2.8 | 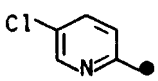 | H | H | H | H | |
| 2.9 | 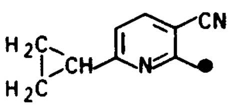 | H | H | H | H | |
| 2.10 | 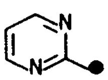 | H | H | H | H | |
| 2.11 | 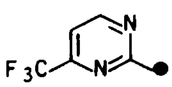 | H | H | H | H | |
| 2.12 | 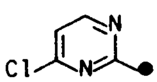 | H | H | H | H | |

Tabelle 2 (Fortsetzung)

| Verbindung Nr. | A | $R^1$ | $R^6$ | $R^7$ | $R^8$ | phys. Daten |
|---|---|---|---|---|---|---|
| 2.13 | 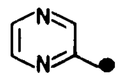 | H | H | H | H | |
| 2.14 | 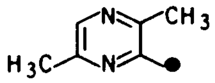 | H | H | H | H | |
| 2.15 | 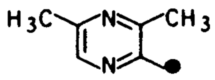 | H | H | H | H | |
| 2.16 | 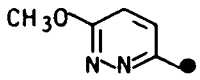 | H | H | H | H | |
| 2.17 | 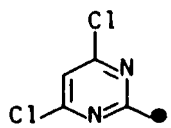 | H | H | H | H | |
| 2.18 | 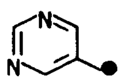 | H | H | H | H | |
| 2.19 | 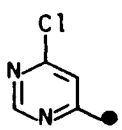 | H | H | H | H | |

Tabelle 3

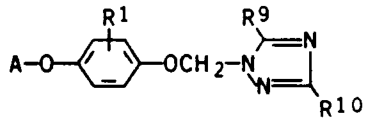

(Iac)

| Verbindung Nr. | A | $R^1$ | $R^9 = R^{10}$ | phys. Daten Fp. [°C] |
|---|---|---|---|---|
| 3.1 | 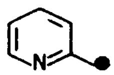 | H | H | 85 - 92 |
| 3.2 | 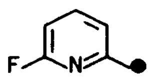 | H | H | 85 - 93 |
| 3.3 | 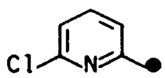 | H | H | 103 - 106 |
| 3.4 | 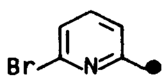 | H | H | 128 - 130 |
| 3.5 | 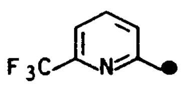 | H | H | 92 - 95 |
| 3.6 | 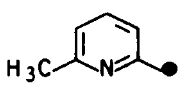 | H | H | 83 - 85 |
| 3.7 | 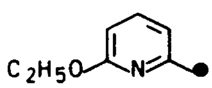 | H | H | |
| 3.8 | 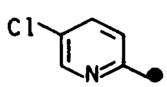 | H | H | 98 - 100 |
| 3.9 | 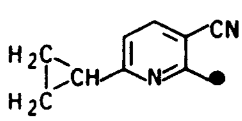 | H | H | 90 - 94 |
| 3.10 | 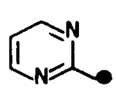 | H | H | 160 - 163 |
| 3.11 | 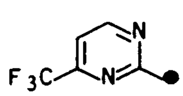 | H | H | |

Tabelle 3 (Fortsetzung)

| Verbindung Nr. | A | $R^1$ | $R^9 = R^{10}$ | phys. Daten Fp. [°C] |
|---|---|---|---|---|
| 3.12 | 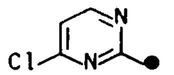 | H | H | |
| 3.13 | 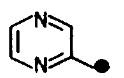 | H | H | 88 - 94 |
| 3.14 | 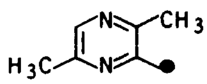 | H | H | |
| 3.15 | 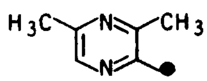 | H | H | 84 - 88 |
| 3.16 | 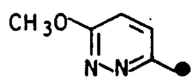 | H | H | |
| 3.17 | 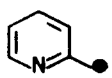 | H | $CH_3$ | |
| 3.18 | 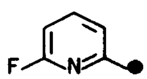 | H | $CH_3$ | |
| 3.19 | 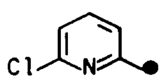 | H | $CH_3$ | |
| 3.20 | 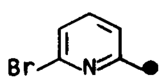 | H | $CH_3$ | |
| 3.21 | 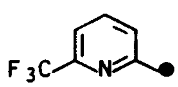 | H | $CH_3$ | |
| 3.22 | 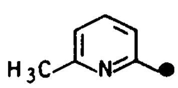 | H | $CH_3$ | |
| 3.23 | 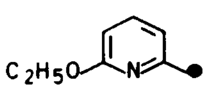 | H | $CH_3$ | |
| 3.24 | 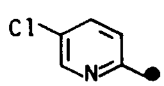 | H | $CH_3$ | |

Tabelle 3 (Fortsetzung)

| Verbindung Nr. | A | $R^1$ | $R^9 = R^{10}$ | phys. Daten Fp. [°C] |
|---|---|---|---|---|
| 3.25 | 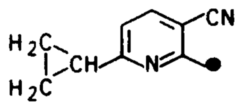 | H | $CH_3$ | |
| 3.26 | 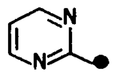 | H | $CH_3$ | |
| 3.27 | 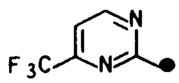 | H | $CH_3$ | |
| 3.28 | 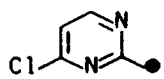 | H | $CH_3$ | |
| 3.29 | 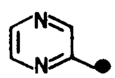 | H | $CH_3$ | |
| 3.30 | 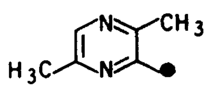 | H | $CH_3$ | |
| 3.31 | 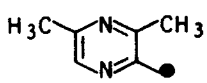 | H | $CH_3$ | |
| 3.32 | 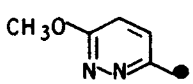 | H | $CH_3$ | |
| 3.33 | 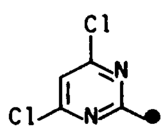 | H | H | |
| 3.34 | 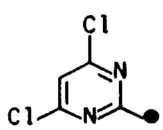 | H | $CH_3$ | |
| 3.35 | 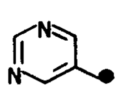 | H | H | |
| 3.36 | 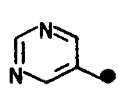 | H | $CH_3$ | |

Tabelle 3 (Fortsetzung)

| Verbindung Nr. | A | $R^1$ | $R^9 = R^{10}$ | phys. Daten Fp. [°C] |
|---|---|---|---|---|
| 3.37 | 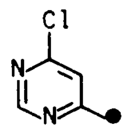 | H | H | |
| 3.38 | 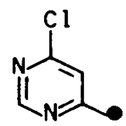 | H | $CH_3$ | |

Tabelle 4

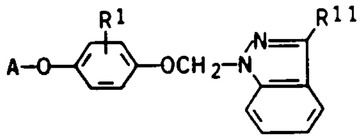

(Iad)

| Verbindung Nr. | A | $R^1$ | $R^{11}$ | phys. Daten |
|---|---|---|---|---|
| 4.1 | 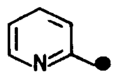 | H | H | |
| 4.2 | 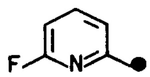 | H | H | |
| 4.3 | 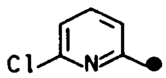 | H | H | |
| 4.4 | 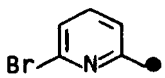 | H | H | |
| 4.5 | 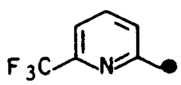 | H | H | |
| 4.6 | 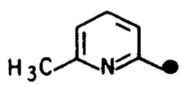 | H | H | |
| 4.7 | 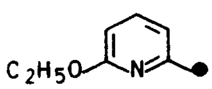 | H | H | |
| 4.8 | 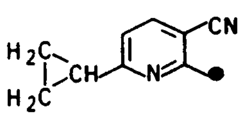 | H | H | |
| 4.9 | 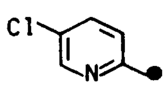 | H | H | |
| 4.10 | 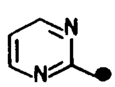 | H | H | |
| 4.11 | 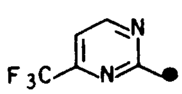 | H | H | |

Tabelle 4 (Fortsetzung)

| Verbindung Nr. | A | $R^1$ | $R^9 = R^{10}$ | phys. Daten |
|---|---|---|---|---|
| 4.12 | 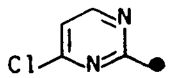 | H | H | |
| 4.13 | 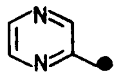 | H | H | |
| 4.14 | 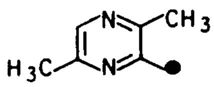 | H | H | |
| 4.15 | 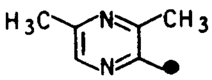 | H | H | |
| 4.16 | 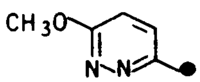 | H | H | |
| 4.17 | 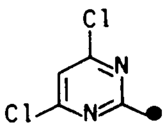 | H | H | |
| 4.18 | 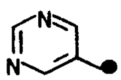 | H | H | |
| 4.19 | 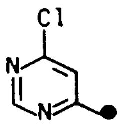 | H | H | |

Tabelle 5

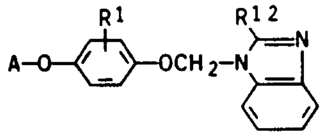

(Iae)

| Verbindung Nr. | A | $R^1$ | $R^{12}$ | phys. Daten |
|---|---|---|---|---|
| 5.1 | 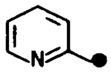 | H | H | |
| 5.2 | 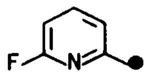 | H | H | |
| 5.3 | 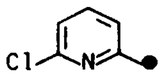 | H | H | |
| 5.4 | 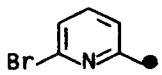 | H | H | |
| 5.5 | 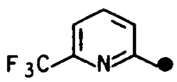 | H | H | |
| 5.6 | 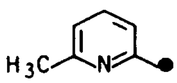 | H | H | |
| 5.7 | 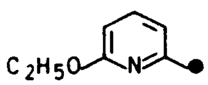 | H | H | |
| 5.8 | 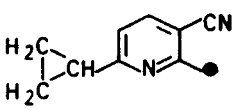 | H | H | |
| 5.9 | 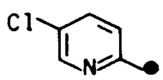 | H | H | |
| 5.10 | 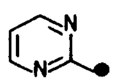 | H | H | |
| 5.11 | 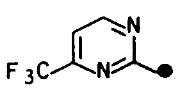 | H | H | |

Tabelle 5 (Fortsetzung)

| Verbindung Nr. | A | $R^1$ | $R^{12}$ | phys. Daten |
|---|---|---|---|---|
| 5.12 | 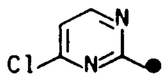 | H | H | |
| 5.13 | 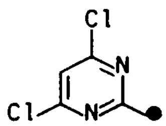 | H | H | |
| 5.14 | 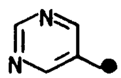 | H | H | |
| 5.16 | 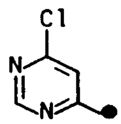 | H | H | |
| 5.17 | 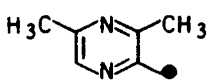 | H | H | |
| 5.18 | 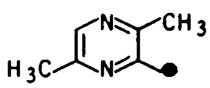 | H | H | |
| 5.19 | 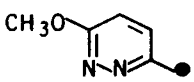 | H | H | |
| 5.20 | 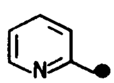 | H | $CH_3$ | |
| 5.21 | 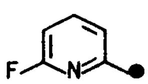 | H | $CH_3$ | |
| 5.22 | 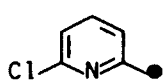 | H | $CH_3$ | |
| 5.23 | 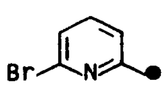 | H | $CH_3$ | |
| 5.24 | 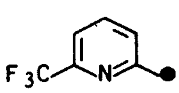 | H | $CH_3$ | |

Tabelle 5 (Fortsetzung)

| Verbindung Nr. | A | $R^1$ | $R^{12}$ | phys. Daten |
|---|---|---|---|---|
| 5.25 | 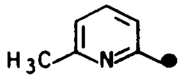 | H | $CH_3$ | |
| 5.26 | 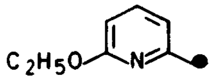 | H | $CH_3$ | |
| 5.27 | 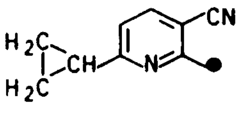 | H | $CH_3$ | |
| 5.28 | 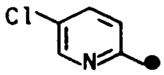 | H | $CH_3$ | |
| 5.29 | 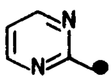 | H | H | |
| 5.30 | 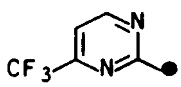 | H | H | |
| 5.31 | 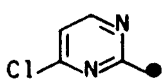 | H | H | |
| 5.32 | 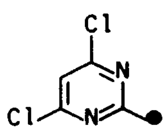 | H | $CH_3$ | |
| 5.33 | 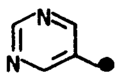 | H | $CH_3$ | |
| 5.34 | 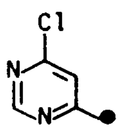 | H | $CH_3$ | |
| 5.35 | 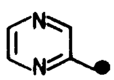 | H | $CH_3$ | |
| 5.36 | 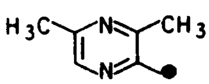 | H | $CH_3$ | |

Tabelle 5 (Fortsetzung)

| Verbindung Nr. | A | R1 | R12 | phys. Daten |
|---|---|---|---|---|
| 5.37 | 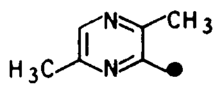 | H | $CH_3$ | |
| 5.38 | 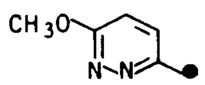 | H | $CH_3$ | |

Beispiel 4

2-[4-(6-Chlor-2-pyridinyloxy)-phenoxymethyl]-5-bromthiophen (Verbindung Nr. 6.3)

3,5 g 4-(6-chlor-2-pyridinyloxy)phenol und 3,2 g Kaliumcarbonat werden in 100 ml trockenem Dimethylformamid 1 Stunde bei 60 °C gerührt. Anschließend werden 6,5 g 5-Brom-2-chlormethylthiophen in 30 ml trockenem Dimethylformamid zugetropft. Man rührt 6 Stunden bei 80 °C und über Nacht bei Raumtemperatur (ca. 20 °C) nach. Zur Aufarbeitung gießt man in 200 ml Eiswasser. Das ausgefallene Rohprodukt wird abgesaugt und aus n-Hexan/Essigester = 4/1 umkristallisiert. Man erhält 3,1 g 2-[4-(6-Chlor-2-pyridinyloxy)-phenoxymethyl]5-bromthiophen mit Fp.: 78 - 80 °C.

Beispiel 5

5-[4-(6-Chlor-2-pyridinyloxy)-phenoxymethyl]-3-cyclopropylisoxazol (Verbindung Nr. 6.22)

Zu 1,3 g 100 %igem Natriumhydrid in 40 ml trockenem Dimethylformamid tropft man bei Raumtemperatur (ca. 20 °C) 11,1 g 4-(6-Chlor-2-pyridinyloxy)phenol in 100 ml trockenem Dimethylformamid. Zur Vervollständigung der exothermen Reaktion (Wasserstoffentwicklung) rührt man 30 Minuten bei 50 °C nach. Anschließend tropft man bei Raumtemperatur (ca. 20 °C) 7,9 g 5-Chlormethyl-3-cyclopropylisoxazol in 30 ml trockenem Dimethylformamid zu. Man rührt 2 Stunden bei 60 °C und über Nacht bei Raumtemperatur (ca. 20 °C) nach. Zur Aufarbeitung gießt man in 200 ml Eiswasser und extrahiert die Lösung mit Methyl-tert.-butylether. Die organische Phase wird anschließend mit Natronlauge, Wasser und gesättigter Kochsalzlösung gewaschen. Nach dem Trocknen über Natriumsulfat wird das Lösungsmittel im Vakuum verdampft. Das Rohprodukt wird aus Methanol umkristallisiert. Man erhält 13,5 g 5-[4-(6-Chlor-2-pyridinyloxy)-phenoxymethyl]3-cyclopropylisoxazol mit Fp.: 128 - 130 °C.

Beispiel 6

5-[4-(6-Chlor-2-pyridinyloxy)-phenoxymethyl]-2-ethoxy-1,3,4-thiadiazol (Verbindung Nr. 6.79)

Zu 0,6 g 100 %igem Natriumhydrid in 20 ml trockenem Dimethylformamid tropft man bei Raumtemperatur (ca. 20 °C) 4,9 g 4-(6-Chlor-2-pyridinyloxy)phenol in 40 ml trockenem Dimethylformamid. Zur Vervollständigung der exothermen Reaktion (Wasserstoffentwicklung) rührt man 30 Minuten bei 50 °C nach. Anschließend tropft man bei Raumtemperatur (ca. 20 °C) 4 g 5-Chlormethyl-2-ethoxy-1,3,4-thiadiazol in 20 ml trockenem Dimethylformamid zu und rührt 2 Stunden bei 60 °C und über Nacht bei Raumtemperatur (ca. 20 °C) nach. Zur Aufarbeitung gießt man in 200 ml Eiswasser. Das ausgefallene Rohprodukt wird abgesaugt und aus Isopropanol umkristallisiert. Man erhält 5,4 g 5-[4-(6-Chlor-2-pyridinyloxy)-phenoxymethyl]-2-ethoxyl,3,4-thiadiazol mit Fp.: 92 °C - 94 °C.

Entsprechend dieser Herstellvorschriften können die in der folgenden Tabelle 6 beschriebenen Verbindungen Ib hergestellt werden. Die Substitutionsstellen an den Heteroaromaten sind jeweils durch einen Punkt gekennzeichnet.

Die ohne Angabe physikalischer Kenngrößen in der nachstehenden Tabelle 6 aufgeführten Verbindungen I können aus entsprechenden Vorprodukten ohne weiteres erhalten werden und lassen eine gleichartige

Wirkung erwarten.

Tabelle 6

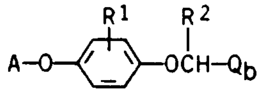

(Ib)

| Verbindung Nr. | A | $R^1$ | $R^2$ | $Q_b$ | phys. Daten/Fp. [°C] $^1$H-NMR in $CDCl_3$, δ [ppm] |
|---|---|---|---|---|---|
| 6.1 | 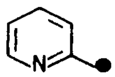 | H | H | 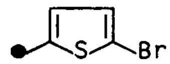 | |
| 6.2 | 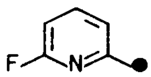 | H | H | 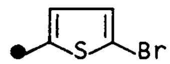 | 74 - 76 |
| 6.3 | 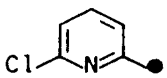 | H | H | 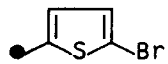 | 78 - 80 |
| 6.4 | 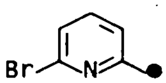 | H | H | 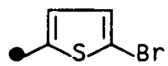 | 71 - 73 |
| 6.5 | 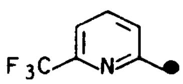 | H | H | 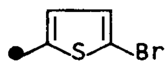 | 70 - 72 |
| 6.6 | 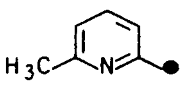 | H | H | 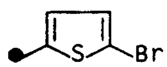 | |
| 6.7 | 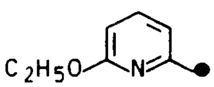 | H | H | 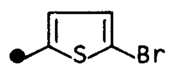 | |
| 6.8 | 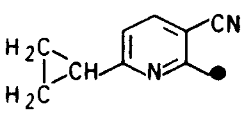 | H | H | 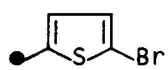 | |
| 6.9 | 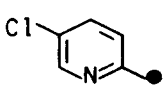 | H | H | 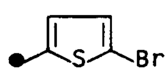 | 98 - 100 |
| 6.10 | 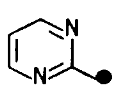 | H | H | 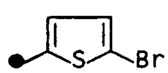 | 103 - 106 |
| 6.11 | 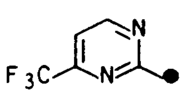 | H | H | 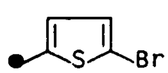 | |

Tabelle 6 - Fortsetzung -

| Verbindung Nr. | A | $R^1$ | $R^2$ | $Q_b$ | phys.Daten/Fp.[°C] $^1$H-NMR in $CDCl_3$, δ [ppm] |
|---|---|---|---|---|---|
| 6.12 | 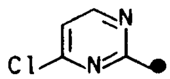 | H | H | 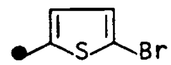 | |
| 6.13 | 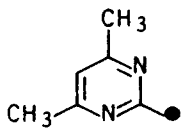 | H | H | 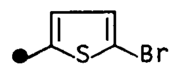 | |
| 6.14 | 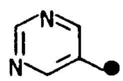 | H | H | 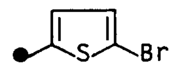 | 86 - 89 |
| 6.15 | 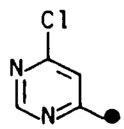 | H | H | 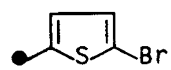 | |
| 6.16 | 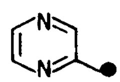 | H | H | 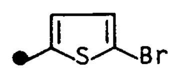 | 120 - 124 |
| 6.17 | 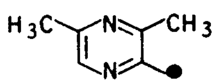 | H | H | 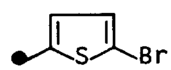 | 101 - 102 |
| 6.18 | 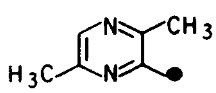 | H | H | 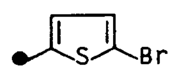 | 91 - 93 |
| 6.19 | 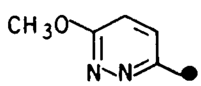 | H | H | 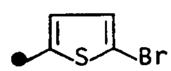 | |
| 6.20 | 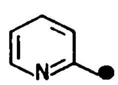 | H | H | 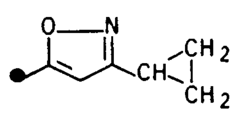 | 68 - 72 |
| 6.21 | 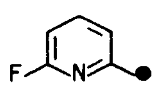 | H | H | 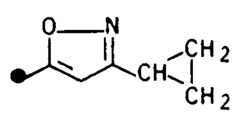 | 99 - 106 |
| 6.22 | 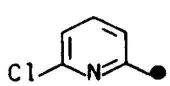 | H | H | 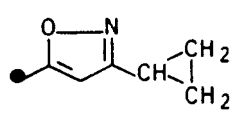 | 128 - 130 |

Tabelle 6 - Fortsetzung -

| Verbindung Nr. | A | $R^1$ | $R^2$ | $Q_b$ | phys.Daten/Fp.[°C] $^1$H-NMR in $CDCl_3$, $\delta$ [ppm] |
|---|---|---|---|---|---|
| 6.23 | 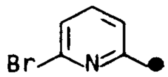 | H | H | 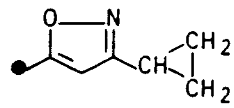 | 127 - 129 |
| 6.24 | 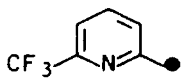 | H | H | 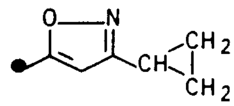 | 134 - 137 |
| 6.25 | 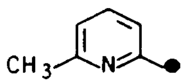 | H | H | 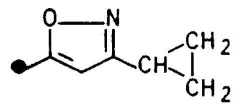 | 97 - 101 |
| 6.26 | 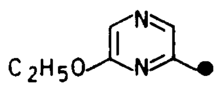 | H | H | 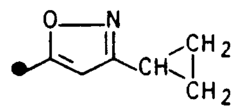 | |
| 6.27 | 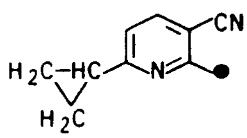 | H | H | 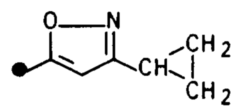 | 140 - 145 |
| 6.28 | 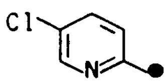 | H | H | 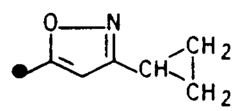 | 116 - 118 |
| 6.29 | 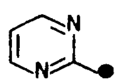 | H | H | 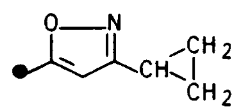 | 119 - 122 |
| 6.30 | 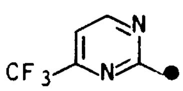 | H | H | 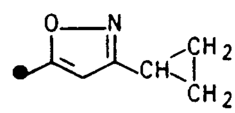 | 175 - 180 |
| 6.31 | 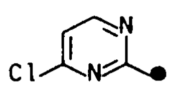 | H | H | 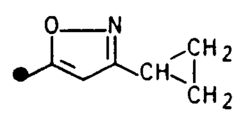 | |
| 6.32 | 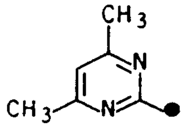 | H | H | 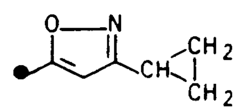 | 81 - 85 |
| 6.33 | 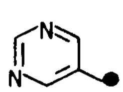 | H | H | 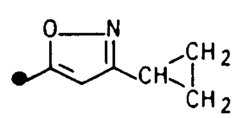 | 83 - 86 |

Tabelle 6 - Fortsetzung -

| Verbindung Nr. | A | $R^1$ | $R^2$ | $Q_b$ | phys.Daten/Fp.[°C] $^1$H-NMR in $CDCl_3$, δ [ppm] |
|---|---|---|---|---|---|
| 6.34 | 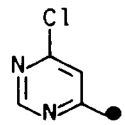 | H | H | 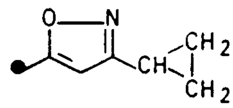 | 83 - 86 |
| 6.35 | 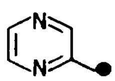 | H | H | 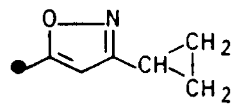 | 113 - 116 |
| 6.36 | 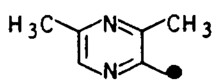 | H | H | 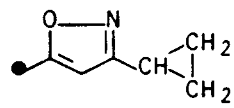 | 94 - 95 |
| 6.37 | 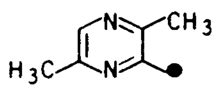 | H | H | 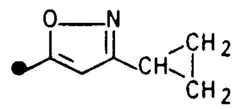 | 94 - 95 |
| 6.38 | 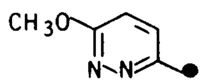 | H | H | 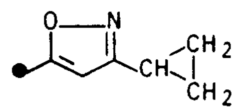 | 129 - 140 |
| 6.39 | 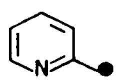 | H | H | 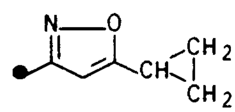 | |
| 6.40 | 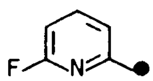 | H | H | 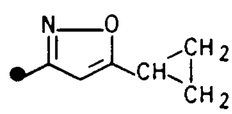 | |
| 6.41 | 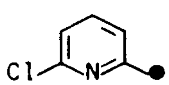 | H | H | 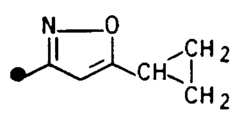 | |
| 6.42 | 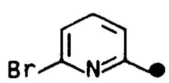 | H | H | 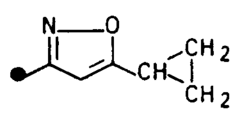 | |
| 6.43 | 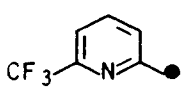 | H | H | 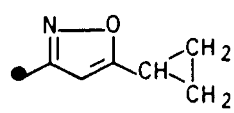 | |
| 6.44 | 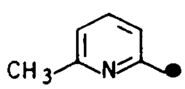 | H | H | 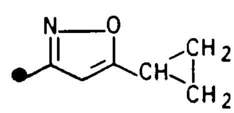 | |

Tabelle 6 - Fortsetzung -

| Verbindung Nr. | A | $R^1$ | $R^2$ | $Q_b$ | phys.Daten/Fp.[°C] $^1$H-NMR in $CDCl_3$, $\delta$ [ppm] |
|---|---|---|---|---|---|
| 6.45 | 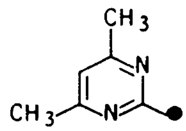 | H | H | 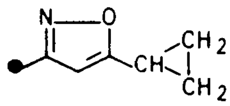 | |
| 6.46 | 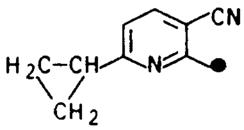 | H | H | 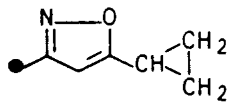 | |
| 6.47 | 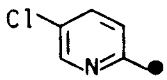 | H | H | 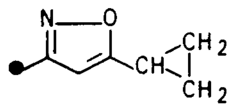 | |
| 6.48 | 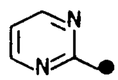 | H | H | 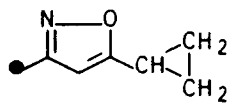 | |
| 6.49 | 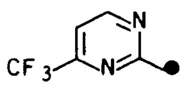 | H | H | 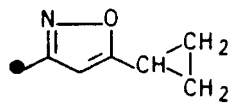 | |
| 6.50 | 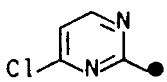 | H | H | 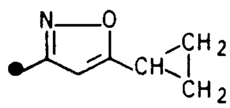 | |
| 6.51 | 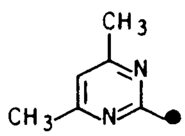 | H | H | 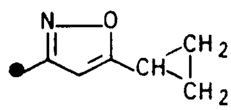 | |
| 6.52 | 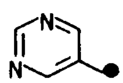 | H | H | 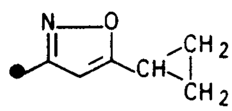 | |
| 6.53 | 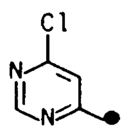 | H | H | 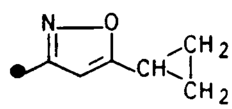 | |
| 6.54 | 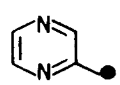 | H | H | 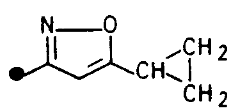 | |

Tabelle 6 - Fortsetzung -

| Verbindung Nr. | A | $R^1$ | $R^2$ | $Q_b$ | phys.Daten/Fp.[°C] $^1H$-NMR in $CDCl_3$, δ [ppm] |
|---|---|---|---|---|---|
| 6.55 | 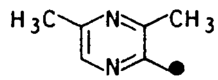 | H | H | 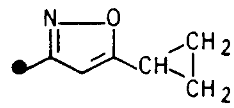 | |
| 6.56 | 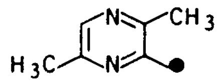 | H | H | 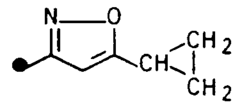 | |
| 6.57 | 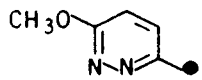 | H | H | 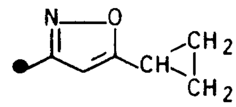 | |
| 6.58 | 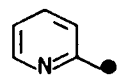 | H | H | 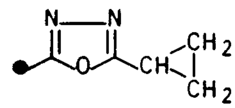 | |
| 6.59 | 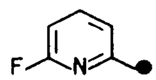 | H | H | 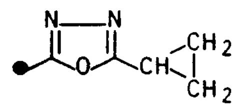 | 80 - 84 |
| 6.60 | 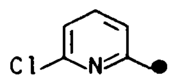 | H | H | 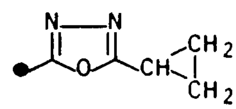 | 200 MHz: 2.15-2.30 (m), 5.23 (s), 7.01-7.21 (m). |
| 6.61 | 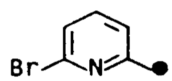 | H | H | 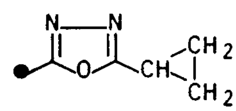 | 250 MHz: 1.08-1.24 (m), 2.12-2.25 (m), 5.20 (s). |
| 6.62 | 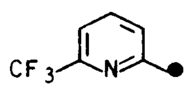 | H | H | 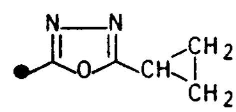 | 250 MHz: 1.15-1.22 (m), 2.13-2.25 (m), 5.19 (s). |
| 6.63 | 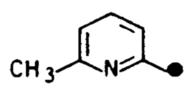 | H | H | 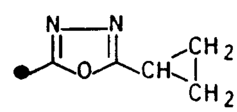 | |
| 6.64 | 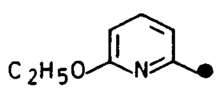 | H | H | 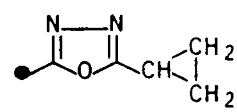 | |
| 6.65 | 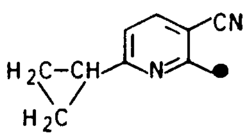 | H | H | 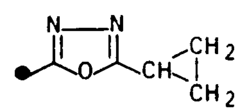 | |

Tabelle 6 - Fortsetzung -

| Verbindung Nr. | A | $R^1$ | $R^2$ | $Q_b$ | phys.Daten/Fp.[°C] $^1H$-NMR in $CDCl_3$, δ [ppm] |
|---|---|---|---|---|---|
| 6.66 | 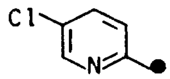 | H | H | 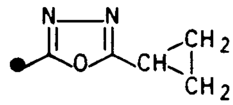 | |
| 6.67 | 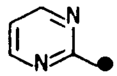 | H | H | 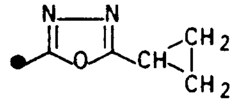 | 300-MHz: 1.05-1.38 (m), 5.21 (s), 6,85 + 7.30 (m) |
| 6.68 | 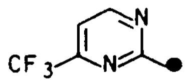 | H | H | 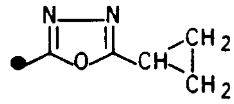 | |
| 6.69 | 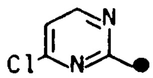 | H | H | 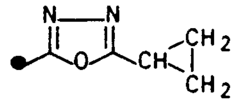 | |
| 6.70 | 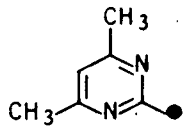 | H | H | 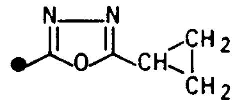 | 125 - 130 |
| 6.71 | 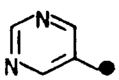 | H | H | 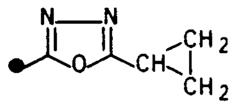 | |
| 6.72 | 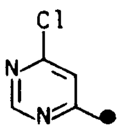 | H | H | 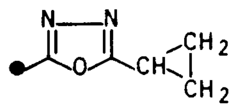 | |
| 6.73 | 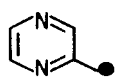 | H | H | 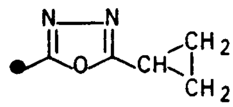 | 51 - 57 |
| 6.74 | 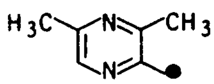 | H | H | 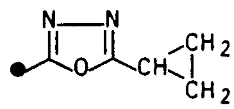 | 78 - 82 |
| 6.75 | 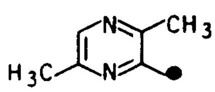 | H | H | 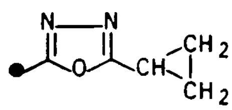 | 125 - 128 |
| 6.76 | 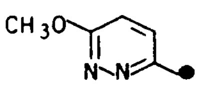 | H | H | 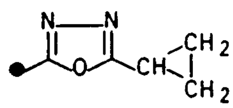 | 47 - 52 |

Tabelle 6 - Fortsetzung -

| Verbindung Nr. | A | $R^1$ | $R^2$ | $Q_b$ | phys.Daten/Fp.[°C] $^1H$-NMR in $CDCl_3$, $\delta$ [ppm] |
|---|---|---|---|---|---|
| 6.77 | 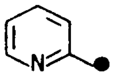 | H | H | 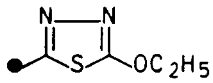 | 55 - 57 |
| 6.78 | 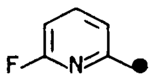 | H | H | 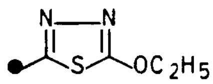 | 79 - 83 |
| 6.79 | 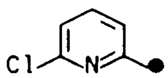 | H | H | 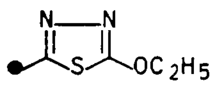 | 92 - 94 |
| 6.80 | 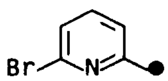 | H | H | 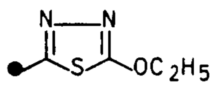 | 80 - 81 |
| 6.81 | 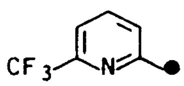 | H | H | 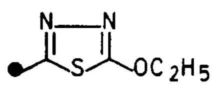 | 90 - 91 |
| 6.82 | 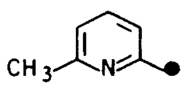 | H | H | 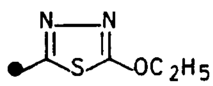 | |
| 6.83 | 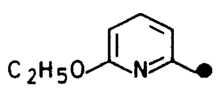 | H | H | 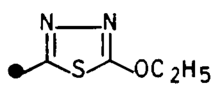 | |
| 6.84 | 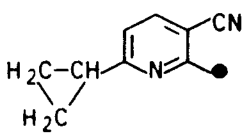 | H | H | 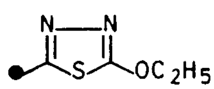 | 128 - 130 |
| 6.85 | 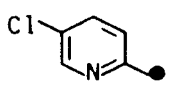 | H | H | 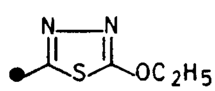 | 93 - 95 |
| 6.86 | 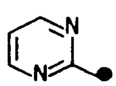 | H | H | 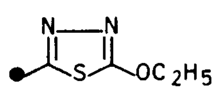 | 92 - 93 |
| 6.87 | 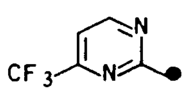 | H | H | 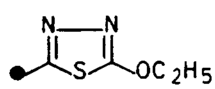 | 105 - 107 |
| 6.88 | 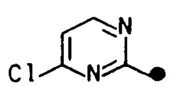 | H | H | 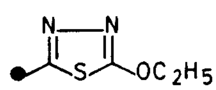 | 121 - 123 |

Tabelle 6 - Fortsetzung -

| Verbindung Nr. | A | $R^1$ | $R^2$ | $Q_b$ | phys.Daten/Fp.[°C] $^1$H-NMR in $CDCl_3$, δ [ppm] |
|---|---|---|---|---|---|
| 6.89 | 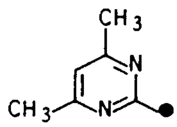 | H | H | 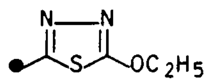 | 97 - 98 |
| 6.90 | 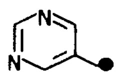 | H | H | 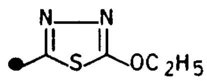 | 86 - 88 |
| 6.91 | 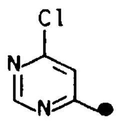 | H | H | 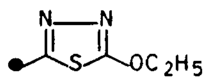 | |
| 6.92 | 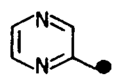 | H | H | 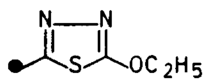 | 86 - 87 |
| 6.93 | 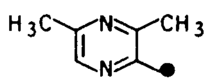 | H | H | 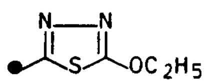 | 100 - 101 |
| 6.94 | 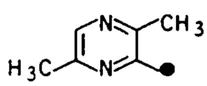 | H | H | 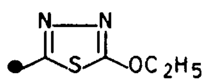 | |
| 6.95 | 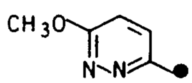 | H | H | 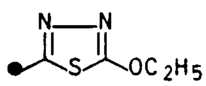 | |
| 6.96 | 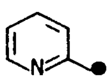 | H | H | 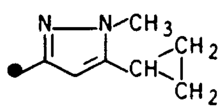 | |
| 6.97 | 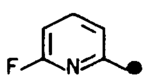 | H | H | 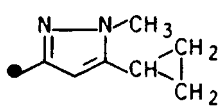 | 66 - 69 |
| 6.98 | 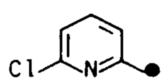 | H | H | 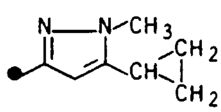 | 89 - 90 |
| 6.99 | 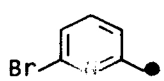 | H | H | 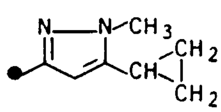 | 90 - 93 |
| 6.100 | 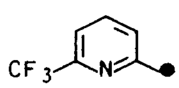 | H | H | 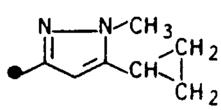 | 53 - 55 |

Tabelle 6 - Fortsetzung -

| Verbindung Nr. | A | $R^1$ | $R^2$ | $Q_b$ | phys.Daten/Fp.[°C] $^1H$-NMR in $CDCl_3$, δ [ppm] |
|---|---|---|---|---|---|
| 6.101 | 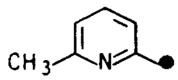 | H | H | 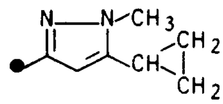 | |
| 6.102 | 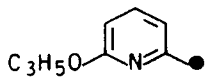 | H | H | 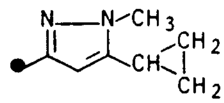 | |
| 6.103 | 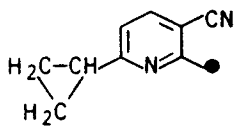 | H | H | 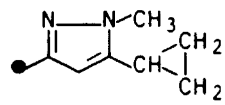 | 117 - 118 |
| 6.104 | 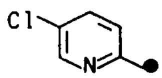 | H | H | 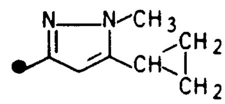 | 65 - 67 |
| 6.105 | 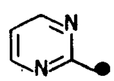 | H | H | 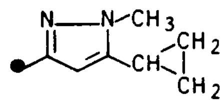 | 100 - 102 |
| 6.106 | 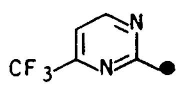 | H | H | 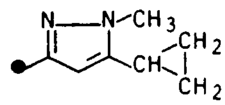 | 69 - 71 |
| 6.107 | 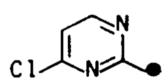 | H | H | 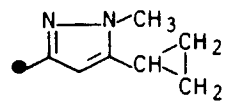 | |
| 6.108 | 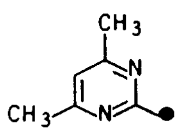 | H | H | 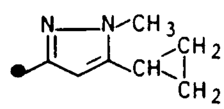 | |
| 6.109 | 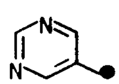 | H | H | 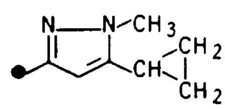 | 37 - 38 |
| 6.110 | 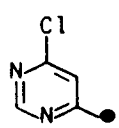 | H | H | 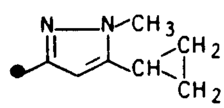 | |
| 6.111 | 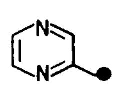 | H | H | 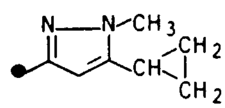 | 88 - 89 |
| 6.112 | 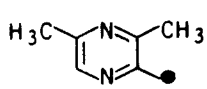 | H | H | 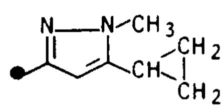 | 82 - 83 |

Tabelle 6 - Fortsetzung -

| Verbindung Nr. | A | $R^1$ | $R^2$ | $Q_b$ | phys.Daten/Fp.[°C] $^1$H-NMR in $CDCl_3$, δ [ppm] |
|---|---|---|---|---|---|
| 6.113 | 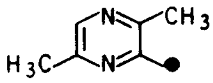 | H | H | 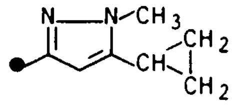 | |
| 6.114 | 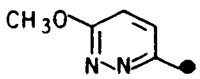 | H | H | 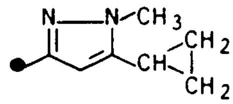 | |
| 6.115 | 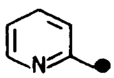 | H | H | 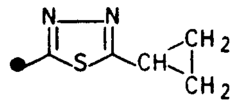 | 72 |
| 6.116 | 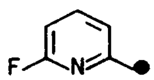 | H | H | 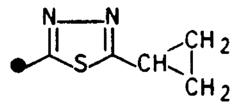 | 92 - 94 |
| 6.117 | 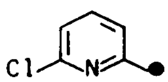 | H | H | 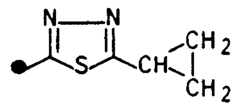 | 76 - 78 |
| 6.118 | 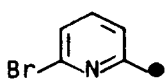 | H | H | 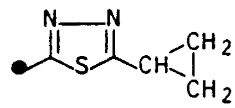 | 69 - 71 |
| 6.119 | 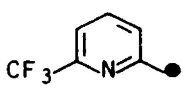 | H | H | 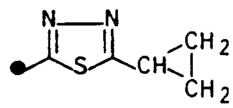 | 80 - 82 |
| 6.120 | 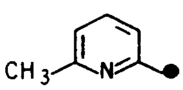 | H | H | 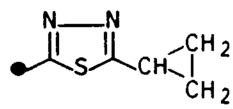 | |
| 6.121 | 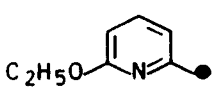 | H | H | 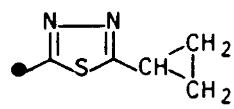 | |
| 6.122 | 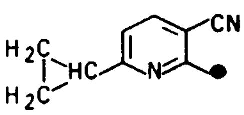 | H | H | 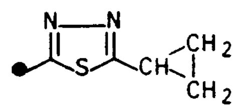 | 136 - 138 |
| 6.123 | 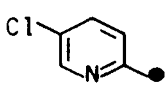 | H | H | 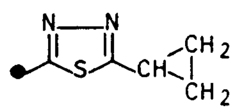 | 90 - 91 |
| 6.124 | 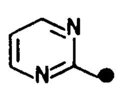 | H | H | 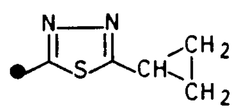 | 93 - 94 |

Tabelle 6 - Fortsetzung -

| Verbindung Nr. | A | $R^1$ | $R^2$ | $Q_b$ | phys.Daten/Fp.[°C] $^1H$-NMR in $CDCl_3$, δ [ppm] |
|---|---|---|---|---|---|
| 6.125 | 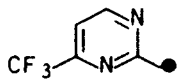 | H | H | 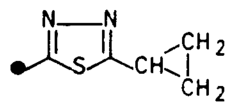 | 130 - 134 |
| 6.126 | 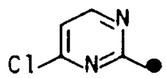 | H | H | 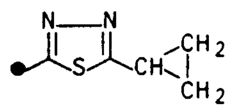 | 97 - 99 |
| 6.127 | 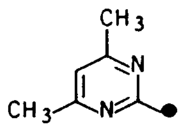 | H | H | 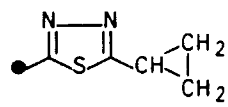 | 110 - 111 |
| 6.128 | 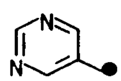 | H | H | 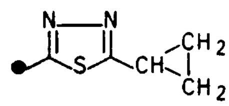 | 74 - 76 |
| 6.129 | 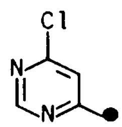 | H | H | 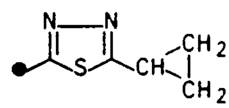 | |
| 6.130 | 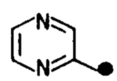 | H | H | 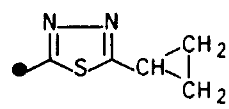 | 99 - 100 |
| 6.131 | 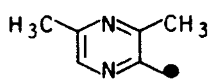 | H | H | 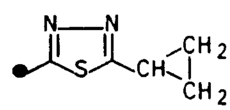 | 107 - 109 |
| 6.132 | 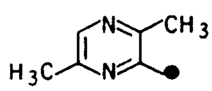 | H | H | 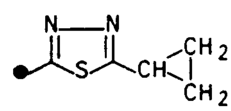 | 86 - 87 |
| 6.133 | 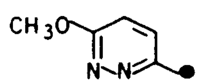 | H | H | 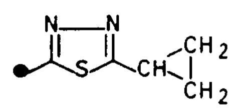 | |
| 6.134 | 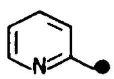 | H | H | 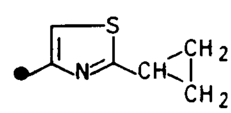 | |
| 6.135 | 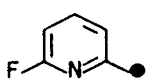 | H | H | 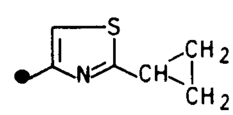 | 68 - 70 |
| 6.136 | 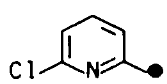 | H | H | 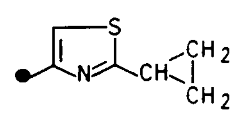 | 63 - 67 |

Tabelle 6 - Fortsetzung -

| Verbindung Nr. | A | $R^1$ | $R^2$ | $Q_b$ | phys.Daten/Fp.[°C] $^1$H-NMR in $CDCl_3$, δ [ppm] |
|---|---|---|---|---|---|
| 6.137 | 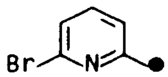 | H | H | 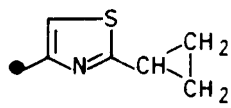 | 52 - 54 |
| 6.138 | 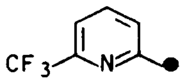 | H | H | 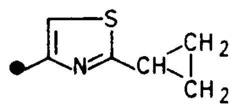 | 63 - 65 |
| 6.139 | 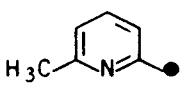 | H | H | 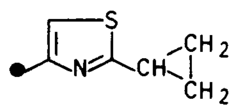 | 73 - 75 |
| 6.140 | 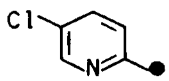 | H | H | 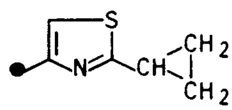 | 61 - 66 |
| 6.141 | 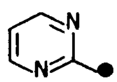 | H | H | 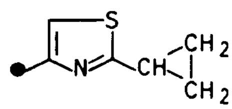 | |
| 6.142 | 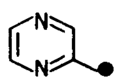 | H | H | 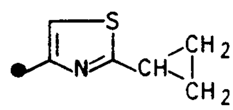 | |
| 6.143 | 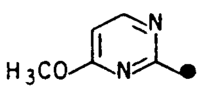 | H | H | 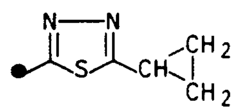 | 85 - 87 |

Anwendungsbeispiele

In den folgenden Beispielen wurden die erfindungsgemäßen Verbindungen bzw. sie enthaltende Mittel hinsichtlich ihrer Wirkung auf Schädlinge mit den nachstehenden Verbindungen des Standes der Technik verglichen:

A 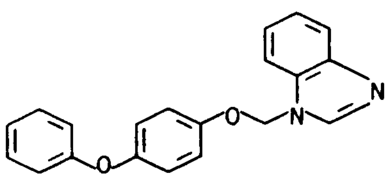 bekannt aus EP 132 606 Beispiel 1

B 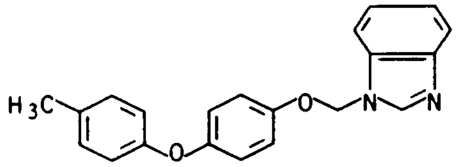 bekannt aus EP-A 132 606 Beispiel 8

Die Reinheit der Vergleichssubstanzen entsprach derjenigen der erfindungsgemäßen Verbindungen und lag bei mindestens 90 bis 95 %. Die Konzentrationen, bei denen die untersuchten Verbindungen eine 100 %ige Mortalität bzw. Hemmung bewirken, sind die jeweiligen Minimalkonzentrationen. Bei jeder Konzentration

werden mindestens zwei Versuche durchgeführt.

Beispiel A

Dysdercus intermedius (Baumwollwanze), ovizide Wirkung

Stecketiketten wurden am oberen Rand mit Klebestreifenstücken (ca. 0,8 cm) beklebt. 24 Stunden vor Versuchsbeginn wurden die Eier durch Eintauchen in das "Sammelgefäß" an dem Klebestreifenrand befestigt.

Die Eier wurden für ca. 5 Sekunden in die wäßrige Wirkstoffaufbereitung getaucht, anschließend ließ man auf Filterpapier abtropfen. Dabei sollten die Eier nicht auf das Papier gelegt werden.

Die zurechtgeschnittenen Etiketten wurden in Plastikpaletten gestellt, Klebestreifen nach oben. Eine halbe mit Wasser durchfeuchtete Watterolle wurde in den Becher gelegt, um Austrocknung zu vermeiden und die Palette mit einer Glasplatte abgedeckt.

Nach ca. 8 Tagen wurde bonitiert. (Bei der Kontrolle müssen die Larven geschlüpft sein).
Beurteilt wurde die Schlupfhemmung in %.

Bei diesem Versuch wurde mit den Verbindungen 1.2, 1.3, 6.60 und 6.79 eine Schlupfhemmung von 80 bis 100 % bei Wirkstoffkonzentrationen von 400 ppm und darunter erzielt, während die Vergleichsverbindungen A und B bei 1000 ppm keine Schlupfhemmung bewirkten.

Beispiel B

Prodenia litura (ägypt. Baumwollwurm); Zucht

Die Zucht erfolgt auf einem Nährboden folgender Zusammensetzung:
515 g Maismehl
130 g Weizenkeime
137 g Bierhefe
18 g Ascorbinsäure
10 g Cellulosepulver
5 g Nipagin
5 g Sorbinsäure
20 g Wessons Salz
2 g Vitamine (Lutavit blend)
5 ml Chloramphenical 0,1 %ig in Ethanol
80 g Agrar
3100 ml Wasser
100 g des Nährbodens werden in 250 ml Becher gefüllt und warm mit der wäßrigen Wirkstoffaufbereitung sorgfältig gemischt. Nach dem Erkalten belegt man jedes Gefäß mit 5 Raupen im 4. Larvenstadium und lagert sie bei 23 °C. Beurteilt wird die Mortalitätsrate nach dem Schlüpfen der Falter in der unbehandelten Kontrolle.

Bei diesem Versuch wurde eine Mortalität von 80 bis 100 % mit 0,04 ppm der Verbindungen 6.33 und 6.24 erzielt. Die Vergleichsverbindungen A und B zeigten bei 1 ppm keine Wirkung.

## Patentansprüche

**1.** Phenoxyalkylsubstituierte Heteroaromaten der allgemeinen Formeln Ia und Ib

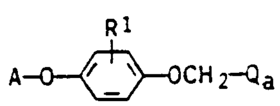

(Ia),

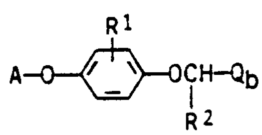

(Ib),

in denen die Substituenten die folgende Bedeutung haben:

A ein gegebenenfalls substituierter heteroaromatischer Rest der Formeln Va bis Ve

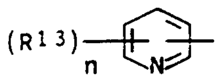
(Va)

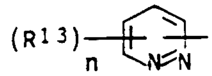
(Vb)

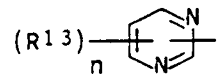
(Vc)

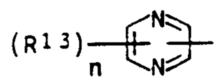
(Vd)

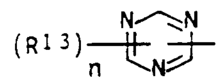
(Ve)

in denen

$R^{13}$ gleich oder verschieden ist und für Wasserstoff, Halogen, $C_1$-$C_4$-Alkyl, $C_1$-$C_3$-Halogenalkyl, $C_1$-$C_4$ -Alkoxy, $C_1$-$C_3$-Halogenalkoxy, $C_3$-$C_6$-Cycloalkyl, Cyano oder Nitro steht und

n eine Zahl von 1 bis 4 bedeutet;

$R^1$ Wasserstoff, Halogen oder $C_1$-$C_3$-Alkyl;

$R^2$ Wasserstoff oder $C_1$-$C_4$-Alkyl;

$Q_a$ ein unsubstituierter oder substituierter Azolrest der Formeln IIa bis IIe

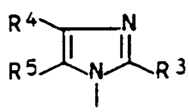
(IIa)

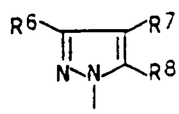
(IIb)

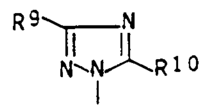
(IIc)

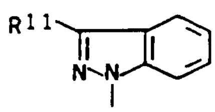
(IId)

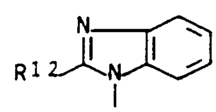
(IIe)

in denen

$R^3$ bis $R^{12}$ Wasserstoff, Halogen, $C_1$-$C_8$-Alkyl, $C_1$-$C_4$-Halogenalkyl, $C_1$-$C_4$-Alkoxy, $C_3$-$C_6$-Cycloalkyl oder Phenyl, 1-Naphthyl oder 2-Naphthyl, welches gegebenenfalls ein- bis dreifach durch Halogen, $C_1$-$C_8$-Alkyl, $C_1$-$C_8$-Alkoxy, $C_1$-$C_4$-Halogenalkyl, $C_1$-$C_3$-Halogenalkoxy substituiert ist, bedeuten;

$Q_b$ ein gegebenenfalls substituierter Fünfringheteroaromat ausgewählt aus der Gruppe Thien-2-yl, Thien-3-yl, Thiazol-2-yl, Thiazol-4-yl, Thiazol-5-yl, Oxazol-2-yl, Oxazol-4-yl, Oxazol-5-yl, Imidazol-2-yl, Imidazol-4-yl, Imidazol-5-yl, Isothiazol-3-yl, Isothiazol-4-yl, Isothiazol-5-yl, Pyrazol-3-yl, Pyrazol-4-yl, Isoxazol-3-yl, Isoxazol-5-yl, 1,3,4-Thiadiazol-2-yl, 1,3,4-Oxadiazol-2-yl, 1,2,4-Triazol-3-yl und 1,2,4-Thiadiazol-3-yl, wobei dieser Rest ein- oder mehrfach durch Halogen, $C_1$-$C_8$-Alkyl, $C_2$-$C_8$-Alkenyl, $C_1$-$C_4$-Halogenalkyl, $C_1$-$C_8$-Alkloxy, $C_1$-$C_8$-Alkylthio, $C_2$-$C_8$-Alkoxyalkyl oder $C_3$-$C_8$-Cycloalkyl substituiert sein kann,

ausgenommen diejenigen Verbindungen Ib, in denen $A_2$ für gegebenenfalls substituiertes Pyridin-2-yl steht, wenn R Methyl und $Q_b$ ein 5-Alkoxy-substituiertes 1,3,4-Thiadiazol-2-yl bedeutet.

**2.** Verfahren zur Herstellung der phenoxyalkylsubstituierten Heteroaromaten der allgemeinenen Formeln Ia und Ib gemäß Anspruch 1, dadurch gekennzeichnet, daß man in an sich bekannter Weise Phenole der

Formel III

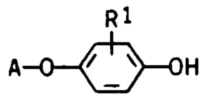

(III)

mit einem substituierten Heteroaromaten der Formeln IVa bzw. IVb

$Y\text{-}CH_2\text{-}Q_a$ (IVa)

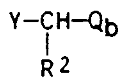

(IVb)

in denen Y eine Abgangsgruppe bedeutet, in Gegenwart einer Base oder das Phenolatanion von III direkt mit IVa bzw. IVb umsetzt.

**3.** Schädlingsbekämpfungsmittel, enthaltend einen phenoxyalkylsubstituierten Heteroaromaten der Formeln Ia oder Ib gemäß Anspruch 1 neben hierfür üblichen Trägerstoffen.

**4.** Schädlingsbekämpfungsmittel nach Anspruch 3, dadurch gekennzeichnet, daß es 0,1 bis 95 Gew.% eines phenoxyalkylsubstituierten Heteroaromaten der Formeln Ia oder Ib enthält.

**5.** Verfahren zur Bekämpfung von Schädlingen, dadurch gekennzeichnet, daß man die Schädlinge und/oder die von Schädlingen freizuhaltenden Flächen und/oder Räume mit einer für Schädlinge wirksamen Menge eines phenoxyalkylsubstituierten Heteroaromaten der Formeln Ia oder Ib gemäß Anspruch 1 behandelt.

**Claims**

**1.** A phenoxyalkyl-substituted heteroaromatic of the general formula Ia or Ib

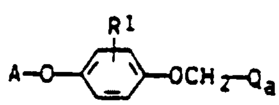

(Ia),

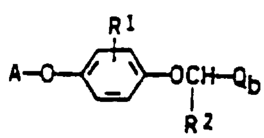

(Ib),

where A is an unsubstituted or substituted heteroaromatic radical of the formulae Va to Ve

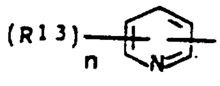
(Va)

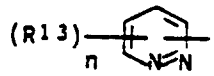
(Vb)

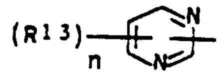
(Vc)

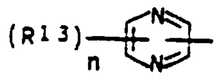
(Vd)

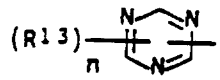
(Ve)

where the radicals $R^{13}$ are identical or different and are each hydrogen, halogen, $C_1$-$C_4$-alkyl, $C_1$-$C_3$-haloalkyl, $C_1$-$C_4$-alkoxy, $C_1$-$C_3$-haloalkoxy, $C_3$-$C_6$-cycloalkyl, cyano or nitro and n is from 1 to 4,
$R^1$ is hydrogen, halogen or $C_1$-$C_3$-alkyl, $R^2$ is hydrogen or $C_1$-$C_4$-alkyl, $Q_a$ is an unsubstituted or substituted azole radical of the formulae IIa to IIe

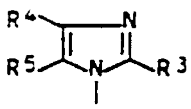
(IIa)

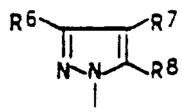
(IIb)

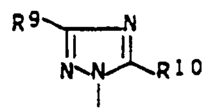
(IIc)

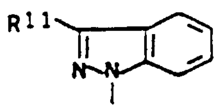
(IId)

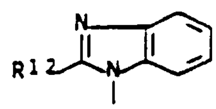
(IIe)

where $R^3$ to $R^{12}$ are each hydrogen, halogen, $C_1$-$C_8$-alkyl, $C_1$-$C_4$-haloalkyl, $C_1$-$C_4$-alkoxy, $C_3$-$C_6$-cycloalkyl or phenyl, 1-naphthyl or 2-naphthyl which is unsubstituted or monosubstituted to trisubstituted by halogen, $C_1$-$C_8$-alkyl, $C_1$-$C_8$-alkoxy, $C_1$-$C_4$-haloalkyl or $C_1$-$C_3$-haloalkoxy;
and Qb is an unsubstituted or substituted heteroaromatic radical which has a five-membered ring and is selected from the group consisting of thien-2-yl, thien-3-yl, thiazol-2-yl, thiazol-4-yl, thiazol-5-yl, oxazol-2-yl, oxazol-4-yl, oxazol-5-yl, imidazol-2yl, imidazol-4-yl, imidazol-5-yl, isothiazol-3-yl, isothiazol-4-yl, isothiazol-5-yl, pyrazol-3-yl, pyrazol-4-yl, isoxazol-3-yl, isoxazol-5-yl, 1,3,4-thiadiazol-2-yl, 1,3,4-oxadiazol-2-yl, 1,2,4-triazol-3-yl and 1,2,4-thiadiazol-3-yl, it being possible for this radical to be monosubstituted or polysubstituted by halogen, $C_1$-$C_8$-alkyl, $C_2$-$C_8$-alkenyl, $C_1$-$C_4$-haloalkyl, $C_1$-$C_8$-alkoxy, $C_1$-$C_8$-alkylthio, $C_2$-$C_8$-alkoxyalkyl or $C_3$-$C_8$-cycloalkyl, with the exception of those compounds Ib where $A_2$ is substituted or unsubstituted pyridin-2-yl when $R^2$ is methyl and $Q_b$ is a 5-alkoxy-substituted 1,3,4-thiadiazol-2-yl.

**2.** A process for the preparation of a phenoxyalkyl-substituted heteroaromatic of the general formula Ia or Ib as claimed in claim 1, wherein a phenol of the formula III

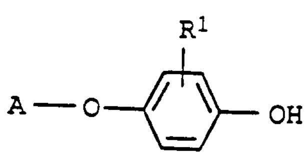
(III)

is reacted in a conventional manner with a substituted heteroaromatic of the formula IVa or IVb

$Y$-$CH_2$-$Q_a$ (IVa)

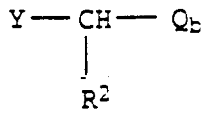

(IVb)

where Y is a leaving group, in the presence of a base, or the phenolate anion of III is reacted directly with IVa or IVb.

3. A pesticide containing a phenoxyalkyl-substituted heteroaromatic of the formula Ia or Ib as claimed in claim 1, in addition to customary carriers.

4. A pesticide as claimed in claim 3, which contains from 0.1 to 95% by weight of a phenoxyalkyl-substituted heteroaromatic of the formula Ia or Ib.

5. A method for controlling pests, wherein the pests and/or the areas and/or spaces to be kept free from pests are treated with a pesticidally effective amount of a phenoxyalkyl-substituted heteroaromatic of the formula Ia or Ib as claimed in claim 1.

## Revendications

1. Hétéroaromates substitués par phénoxyalkyle des formules générales Ia et Ib

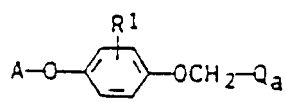

(Ia),

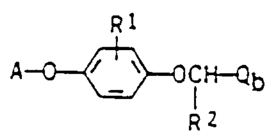

(Ib),

dans lesquelles les substituants ont la signification suivantes :
A un reste hétéroaromatique éventuellement substitué des formules Va à Ve

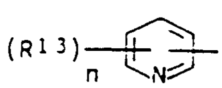

(Va)

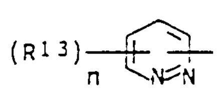

(Vb)

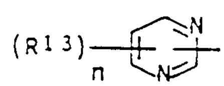

(Vc)

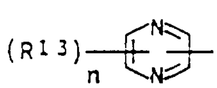

(Vd)

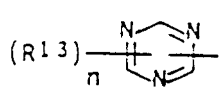

(Ve)

dans lesquelles
$R^{13}$ est le même ou différent et est mis pour hydrogène, halogène, alkyle en $C_1$-$C_4$, halogénoalkyle en $C_1$-$C_3$, alcoxy en $C_1$-$C_4$, halogénoalcoxy en $C_1$-$C_3$, cycloalkyle en $C_3$-$C_6$, cyano ou nitro, et
n est un nombre de 1 à 4.
$R^1$ est hydrogène, halogène ou alkyle en $C_1$-$C_3$
$R^2$ est hydrogène ou alkyle en $C_1$-$C_4$
$Q_a$ est un reste azole substitué ou non des formules IIa à IIe

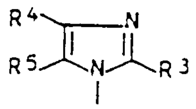
(IIa)

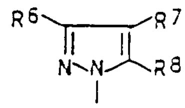
(IIb)

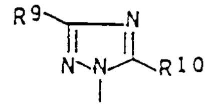
(IIc)

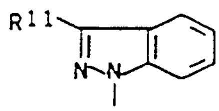
(IId)

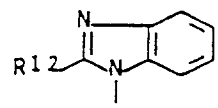
(IIe)

dans lesquelles
$R^3$ à $R^{12}$ représentent hydrogène, halogène, alkyle en $C_1$-$C_8$, halogénoalkyle en $C_1$-$C_4$, alcoxy en $C_1$-$C_4$, cycloalkyle en $C_3$-$C_6$ ou
phényle, 1-naphtyle ou 2-naphtyle, qui est éventuellement substitué une à trois fois par halogène, alkyle en $C_1$-$C_8$, alcoxy en $C_1$-$C_8$, halogénoalkyle en $C_1$-$C_4$, halogénoalcoxy en $C_1$-$C_3$ ;
$Q_b$ est un hétéroaromate à noyau pentagonal éventuellement substitué, choisi dans le groupe thièn-2-yle, thièn-3-yle, thiènazol-2-yle, thiazol-4-yle, thiazol-5-yle, oxazol-2-yle, oxazol-4-yle, oxazol-5-yle, imidazol-2-yle, imidazol-4-yle, imidazol-5-yle, isothiazol-3-yle, isothiazol-4-yle, isothiazol-5-yle, pyrzol-3-yle, pyrazol-4-yle, isoxazol-3-yle, isoxazol-5-yle, 1,3,4-thiadiazol-2-yle, 1,3,4-oxadiazol-2-yle, 1,2,4-triazol-3-yle et 1,2,4-thiadiazol-3-yle, ce reste pouvant être substitué une ou plusieurs fois par halogène, alkyle en $C_1$-$C_8$, alcényle en $C_2$-$C_8$, halogénalkyle en $C_1$-$C_4$, alcoxy en $C_1$-$C_8$, alkylthio en $C_1$-$C_8$, alcoxyalkyle en $C_2$-$C_8$ ou cycloalkyle en $C_3$-$C_8$,
à l'exception des composés Ib dans lesquels $A_2$ est mis pour pyridin-2-yle éventuellement substitué, quand $R^2$ représente méthyle et $Q_b$ une 1,3,4-thiadiazole-2-yle substitué par 5-alcoxy.

**2.** Procédé de préparation des hétéroaromates substitués par phénoxyalkyle des formules générales Ia et Ib selon la revendication I, caractérisé par le fait que l'on fait réagir, de manière connue en soi, des phénols de formule III

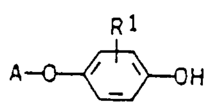
(III)

avec un hétéroaromate substitué des formules IVa ou IVb

$Y$-$CH_2$-$Q_a$ (IVa)

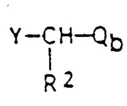
(IVb)

dans lesquelles Y représente un groupe éliminable, en présence d'une base, ou l'anion phénolate de III directement avec IVa ou IVb.

**3.** Pesticides, contenant un hétéroaromate substitué par phénoxyalkyle de formule Ia ou Ib selon la revendication 1 à côté de véhicules usuels pour cela.

**4.** Pesticide selon la revendication 3, caractérisé par le fait qu'il contient 0,1 à 95 % en poids d'un hétéroaromate de formule Ia ou Ib substitué par phénoxyalkyle.

**5.** Procédé de lutte contre les parasites, caractérisé par le fait qu'on traite les parasites et/ou les surfaces et/ou espaces à maintenir exempts des parasites avec une quantité active sur les parasites d'un hétéroaromate substitué par phénoxyalkyle des formules Ia ou Ib selon la revendication 1.